(19) Europäisches Patentamt
European Patent Office
Office européen des brevets

(11) Veröffentlichungsnummer : **0 335 832 B1**

(12) # EUROPÄISCHE PATENTSCHRIFT

(45) Veröffentlichungstag der Patentschrift : **10.02.93 Patentblatt 93/06**

(21) Anmeldenummer : **89810207.4**

(22) Anmeldetag : **16.03.89**

(51) Int. Cl.$^5$ : **C07C 281/18,** C07C 337/06, C07D 213/78, C07D 213/81, C07D 239/28, C07D 401/04, C07D 213/86, C07C 281/06, C07D 213/00, C07D 239/00, C07D 401/00

(54) **Arylhydrazone.**

Die Akte enthält technische Angaben, die nach dem Eingang der Anmeldung eingereicht wurden und die nicht in dieser Patentschrift enthalten sind.

(30) Priorität : **25.03.88 CH 1139/88**

(43) Veröffentlichungstag der Anmeldung : **04.10.89 Patentblatt 89/40**

(45) Bekanntmachung des Hinweises auf die Patenterteilung : **10.02.93 Patentblatt 93/06**

(84) Benannte Vertragsstaaten : **AT BE CH DE ES FR GB GR IT LI LU NL SE**

(56) Entgegenhaltungen :
**CH-A- 496 671**
**US-A- 3 753 680**

(56) Entgegenhaltungen :
**JOURNAL OF MEDICINAL CHEMISTRY, Band 24, Nr. 10, Oktober 1981, Seiten 1181-1184, American Chemical Society; I. ANTONINI et al.: "N*-N*-S* Tridentate ligand system as potential antitumor agents"**

(73) Patentinhaber : **CIBA-GEIGY AG**
**Klybeckstrasse 141**
**CH-4002 Basel (CH)**

(72) Erfinder : **Stanek, Jaroslav, Dr.**
**Hangstrasse 9**
**CH-4144 Arlesheim (CH)**
Erfinder : **Caravatti, Giorgio, Dr.**
**Kurzelängeweg 30**
**CH-4123 Allschwil (CH)**
Erfinder : **Frei, Jörg, Dr.**
**Buechring 36**
**CH-4434 Hölstein (CH)**
Erfinder : **Capraro, Hans-Georg, Dr.**
**Habsburgerstrasse 60**
**CH-4310 Rheinfelden (CH)**

**EP 0 335 832 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Verbindungen der Formel I

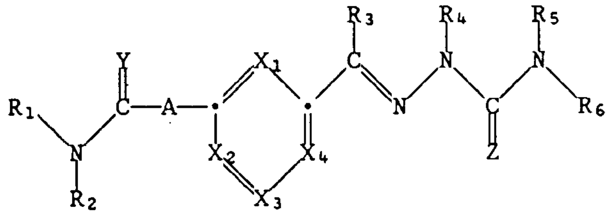

(I),

worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Wasserstoff, Niederalkyl, Hydroxy, verethertes oder verestertes Hydroxy oder unsubstituiertes oder mono- oder disubstituiertes Amino bedeutet; die Reste $R_2$, $R_3$, $R_4$, $R_5$, $R_7$, $R_8$ und $R_9$ unabhängig voneinander Wasserstoff oder Niederalkyl bedeuten, oder $R_1$ und $R_5$ zusammen auch Alkylen bedeuten können; und $R_5$ für Wasserstoff, Niederalkyl, Cycloalkyl, Arylniederalkyl, Aryl, freies oder funktionell abgewandeltes Carboxy, Hydroxy, verethertes oder verestertes Hydroxy, unsubstituiertes oder mono- oder disubstituiertes Amino steht, Tautomere davon, und ihre Salze, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Tautomere können z.B. dann auftreten, wenn Z für $NR_9$ steht und $R_4$ und/oder $R_5$ und/oder $R_5$ Wasserstoff bedeuten:

Der entsprechende Guanylrest, in Formel I als $-N(R_4)-C(=Z)-NR_5R_5$ dargestellt, kann dann z.B. auch in den tautomeren Formen $-N=C(-ZH)-NR_5R_6$, $-N(R_4)-C(-ZH)=NR_6$ oder $-N(R_4)-C(-ZH)=NR_5$ vorliegen.

Ein weiteres Beispiel:

Steht Y für $NR_5$ und ist $R_1$ und/oder $R_2$ Wasserstoff, so kann die entsprechende Amidinstruktur, in Formel I als $-C(=Y)-NR_1R_2$ dargestellt, ebenso in den tautomeren Formen $-C(-YH)=NR_2$ oder $-C(-YH)=NR_1$ auftreten. Dem Fachmann ist das Vorliegen solcher und ähnlicher Tautomere geläufig. Alle diese Tautomere werden von der allgemeinen Formel I mit umfasst.

Die vor- und nachstehend verwendeten Allgemeinbegriffe haben im Rahmen der vorliegenden Anmeldung die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Cycloalkyl enthält z.B. 3 bis 8, vorzugsweise 5 oder 6, Ringkohlenstoffatome und ist z.B. Cyclopropyl, Cyclopentyl, Cyclohexyl oder Cycloheptyl.

Arylniederalkyl ist vorzugsweise Phenylniederalkyl und insbesondere Benzyl.

Aryl ist z.B. Phenyl oder Naphthyl, wie 1- oder 2-Naphthyl. Die Phenyl- oder Naphthylreste können unsubstituiert oder substituiert sein. Aryl ist bevorzugt Phenyl, das unsubstituiert oder durch Niederalkyl, Hydroxy, Niederalkoxy, Halogen, Trifluormethyl und/oder Nitro substituiert ist, und in erster Linie Phenyl.

Freies oder funktionell abgewandeltes Carboxy ist bevorzugt Cyano, ferner auch z.B. Carboxy, verestertes Carboxy, wie z.B. Niederalkoxycarbonyl, oder amidiertes Carboxy, wie z.B. Carbamoyl, N-Niederalkylcarbamoyl oder N,N-Diniederalkylcarbamoyl.

Halogen ist z.B. Fluor oder Iod, insbesondere Brom und vor allem Chlor.

Verethertes Hydroxy ist z.B. Niederalkoxy. Verestertes Hydroxy ist z.B. Niederalkanoyloxy. Monosubstituiertes Amino ist z.B. Niederalkylamino. Disubstituiertes Amino ist z.B. Diniederalkylamino, Niederalkylenamino, z.B. $C_4$-$C_7$- und insbesondere $C_4$-$C_5$-Alkylenamino, z.B. Piperidino, oder Oxa-, Thia- oder Azaniederalkylenamino, z.B. Morpholino, Thiomorpholino, Piperazino oder 4-Niederalkylpiperazino.

Unter der Bedeutung "disubstituiertes Amino" für den Rest $R_5$ ist insbesondere auch eine Gruppe T

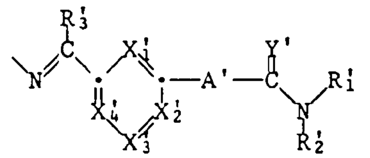

(T)

worin A′, $X_1'$, $X_2'$, $X_3'$, $X_4'$, Y′, $R_1'$, $R_2'$ und $R_3'$ wie die entsprechenden Reste A, $X_1$, $X_2$, $X_3$, $X_4$, Y, $R_1$, $R_2$ und $R_3$ unter Formel I definiert sind, zu verstehen. Bevorzugt sind dabei in einem Rest $R_6$ einer Verbindung der Formel I die Symbole A′, $X_1'$, $X_2'$, $X_3'$, $X_4'$, Y′, $R_1'$, $R_2'$ und $R_3'$ identisch mit den entsprechenden Resten A, $X_1$, $X_2$, $X_3$, $X_4$, Y, $R_1$, $R_2$ und $R_3$, so dass die Verbindungen der Formel I symmetrisch in bezug auf ihre Gruppierung -N($R_4$)-C(=Z)-N($R_5$)- sind.

Eine Gruppe T ist z.B. meta-Amidino-(phenyl oder pyridyl)-methylidenamino und vor allem 3-Amidinophenylmethylidenamino, 2-Amidino-6-pyridylmethylidenamino, 2-Amidino-4-pyridylmethylidenamino, 4-Amidino-2-pyridylmethylidenamino oder 4-Amidino-2-pyrimidylmethylidenamino.

Alkylen gebildet gemeinsam aus den Resten $R_1$ und $R_8$ ist insbesondere $-(CH_2)_2-$ oder $-(CH_2)_3-$ und vor allem $-(CH_2)_2-$. Im letzteren Fall entspricht die Gruppe-C(=$NR_5$)-$NR_1R_2$ ($R_2$ = H) einem 4,5-Dihydroimidazol-2-yl-rest ($\triangleq$ 2-Imidazolinyl).

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder z.B. mit Aminosäuren, wie Arginin oder Lysin, bilden. Bei Anwesenheit von mehreren basischen Gruppen können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Je nach den strukturellen Gegebenheiten können die Verbindungen der vorliegenden Erfindung in Form von Isomerengemischen oder von reinen Isomeren vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym S-Adenosylmethionindecarboxylase (SAMDC). SAMDC spielt als ein Schlüsselenzym eine wichtige Rolle bei der Polyaminsynthese, die in praktisch allen Zellen von Säugetieren, einschliesslich Menschen, abläuft. Durch SAMDC wird die Polyaminkonzentration in der Zelle reguliert. Eine Hemmung des Enzyms SAMDC hat eine Verringerung der Polyaminkonzentration zur Folge. Da eine Verringerung der Polyaminkonzentration eine Hemmung des Zellwachstums bewirkt, ist es möglich, durch Verabreichung von SAMDC-hemmenden Substanzen das Wachstum sowohl von eukaryontischen als auch prokaryontischen Zellen zu hemmen und sogar Zellen abzutöten oder das Einsetzen der Zelldifferenzierung zu hemmen.

Die Hemmung des Enzyms SAMDC kann z.B. mit der Methode von H.G. Williams-Ashmann und A. Schenone, Biochem.Biophys.Res.Communs. 46, 288 (1972) nachgewiesen werden. Die Verbindungen der Erfindung weisen $IC_{50}$-Werte von minimal etwa 0.05 μM auf.

Ein weiterer Vorteil der erfindungsgemässen Verbindungen besteht darin, dass sie nur in geringem Masse im Vergleich zu ihrer starken Hemmwirkung auf SAMDC die Diaminoxidase inhibieren und gut verträglich sind. Die Hemmung der Diaminoxidase ist nach J. Jaenne und D.R. Morris, Biochem. J. 218, 974 (1984) ungünstig, da sie zur Akkumulation von Putrescin und einer indirekten SAMDC-Aktivierung führen kann.

Daher sind die Verbindungen der Formel I z.B. nützlich zur Behandlung benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Die Schweizer Patentschrift Nr. 496 671 erwähnt eine begrenzte Anzahl von Guanylhydrazonen, für die eine Wirkung auf den Kreislauf (Erhöhung der Kontraktionskraft des Herzens, antihypertensive Wirkung und Depletion von Katecholaminspeichern) beschrieben wird. Die Verbindungen unterscheiden sich von denen der Formel I der hier vorliegenden Beschreibung mindestens darin, dass in der Schweizer Patentschrift der Y entsprechende Rest auch dann gleich O ist, wenn in Formel I der Rest A gleich NH ist.

Das US-Patent 3,753,680 beschreibt substituierte Aryliden-semicarbazone, die vom Substituenten

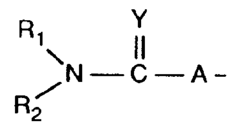

in Verbindungen der Formel I, wie in der hier vorliegenden Anmeldung beschrieben, abweichende Substituenten aufweisen und sich somit deutlich von den Verbindungen der Formel I, wie oben beschrieben, unterscheiden Diese Verbindungen werden im US-Patent als Herbizide beschrieben.

Schliesslich werden von I. Antonini et al. in J. Med. 24, 1181 - 1184 (1981) in Chart I auf Seite 1183 zwei Verbindungen beschrieben (3b, 3c), die von denen der hier vorliegenden Beschreibung mindestens dadurch unterschieden sind, dass der Rest, der in den oben genannten Verbindungen der Formel I Y entspricht, Schwefel anstelle von $NR_8$ oder Sauerstoff bedeutet. Darüberhinaus werden die zwei Einzelverbindungen im genannten Zeitschriftenartikel synthetisiert auf der Suche nach Ribonucleosid-Diphosphat-Reduktasehemmern, die aufgrund ihrer Fähigkeit, das katalytisch wirksame Eisen dieses Enzyms zu chelieren, wirksam sein sollen. Es wird jedoch erwähnt, dass diese Verbindungen selbst unwirksam sind (Seite 1183, linke Spalte, Zeile 15 - 17).

Während in den drei zuletzt genannten Referenzen keine SAMDC-Hemmer beschrieben sind, finden sich in dem oben im Zusammenhang mit der Hemmung von Diaminoxidase genannten Artikel von Jänne et al. (Biochem. J. 218, 947-951 (1984) Methylglyoxal-bis-(guanylhydrazon) und einige Derivate als SAMDC-Hemmer, die sich strukturell sehr deutlich von den Verbindungen der vorliegenden Erfindung unterscheiden. Die dort erwähnten Derivate Ethylglyoxal-bis(guanylhydrazon), Dimethylglyoxal-bis(guanylhydrazon) und N″,N″-Dimethyl-methylglyoxal-bis(guanylhydrazon) haben dabei im Unterschied zu Methylglyoxal-bis(guanylhydrazon) bei der Hemmung von SAMDC bessere Hemmwerte als bei der Hemmung von Diaminoxidase.

Als selektive SAMDC-Hemmer können die Verbindungen der Formel I allein oder auch in Kombination mit anderen pharmakologisch wirksamen Substanzen angewendet werden. Zu denken ist z.B. an eine Kombination mit (a) Inhibitoren anderer Enzyme der Polyaminbiosynthese, z.B. Ornithindecarboxylasehemmern, (b) Inhibitoren der Proteinkinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Bevorzugt sind die Verbindungen der Formel I, worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Wasserstoff, Niederalkyl, Hydroxy, Niederalkoxy oder Amino bedeutet; die Reste $R_2$, $R_3$, $R_4$, $R_5$, $R_7$, $R_8$ und $R_9$ unabhängig voneinander Wasserstoff oder Niederalkyl bedeuten, oder $R_1$ und $R_8$ zusammen auch $-(CH_2)_2-$ oder $-(CH_2)_3-$ bedeuten können; und $R_6$ für Wasserstoff, Niederalkyl, Hydroxy, Amino oder eine Gruppe

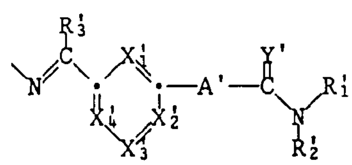

worin A′, $X_1'$, $X_2'$, $X_3'$, $X_4'$, Y′, $R_1'$, $R_2'$ und $R_3'$ wie die entsprechenden Reste A, $X_1$, $X_2$, $X_3$, $X_4$, Y, $R_1$, $R_2$ und $R_3$ definiert sind, steht, Tautomere davon und ihre Salze.

Besonders bevorzugt sind die Verbindungen der Formel I, worin A eine einfache Bindung oder eine Gruppe NH bedeutet; die Reste $X_1$, $X_2$, $X_3$ und $X_4$ folgende Bedeutungen haben:

$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_4 = N, X_1 = X_2 = X_3 = CH$
$X_1 = X_2 = N, X_3 = X_4 = CH$
$X_1 = X_3 = N, X_2 = X_4 = CH$
$X_1 = X_4 = N, X_2 = X_3 = CH$;

Y für $NR_8$ oder auch - falls A eine einfache Bindung bedeutet - für O steht; Z für $NR_9$, O oder S steht; $R_1$ Was-

serstoff, Niederalkyl, Hydroxy, Niederalkoxy oder Amino bedeutet; die Reste $R_2$, $R_3$, $R_8$ und $R_9$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_5$ für Wasserstoff, Niederalkyl, Hydroxy, Amino, 3-Amidinophenylmethylidenamino oder 2-Amidino-6-pyridylmethylidenamino steht, Tautomere davon, und ihre Salze.

In erster Linie bevorzugt sind die Verbindungen der Formel I, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für $NR_5$ oder auch - falls A eine einfache Bindung bedeutet - für O steht; Z für NH oder S steht; $R_1$ Wasserstoff, Niederalkyl, Hydroxy, Niederalkoxy oder Amino bedeutet; die Reste $R_2$, $R_3$ und $R_5$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_5$ für Wasserstoff, Niederalkyl, Hydroxy, Amino, 3-Amidinophenylmethylidenamino oder 2-Amidino-6-pyridylmethylidenamino steht, Tautomere davon, und ihre Salze.

Vor allem bevorzugt sind die Verbindungen der Formel I, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für NH oder auch - falls A eine einfache Bindung bedeutet - für O steht; Z für NH oder S steht; $R_1$ Wasserstoff, Niederalkyl, Hydroxy, Niederalkoxy oder Amino bedeutet; $R_3$ Wasserstoff oder Niederalkyl bedeutet; $R_2$, $R_4$ und $R_5$ Wasserstoff bedeuten und $R_6$ für Wasserstoff, Niederalkyl, Hydroxy oder Amino steht, Tautomere davon, und ihre Salze.

Weiterhin bevorzugt sind die Verbindungen der Formel I, worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_5$ oder auch - falls A eine einfache Bindung bedeutet - für O steht; Z für $NR_9$, O oder S steht; die Reste $R_1$, $R_2$, $R_3$, $R_4$, $R_5$, $R_7$, $R_5$ und $R_9$ unabhängig voneinander Wasserstoff oder Niederalkyl bedeuten und $R_5$ für Wasserstoff, Niederalkyl, Hydroxy, Amino oder 3-Amidinophenylmethylidenamino steht, Tautomere davon, und ihre Salze.

Besonders bevorzugt sind ferner die Verbindungen der Formel I, worin A eine einfache Bindung oder eine Gruppe NH bedeutet; die Reste $X_1$, $X_2$, $X_3$ und $X_4$ folgende Bedeutungen haben:

$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$;

Y für $NR_8$ oder auch - falls A eine einfache Bindung bedeutet - für O steht; Z für $NR_9$, O oder S steht; die Reste $R_1$, $R_2$, $R_3$, $R_8$ und $R_9$ unabhängig voneinander Wasserstoff oder Niederalkyl bedeuten, $R_4$ und $R_5$ Wasserstoff bedeuten und $R_5$ für Wasserstoff, Niederalkyl, Hydroxy, Amino, oder 3-Amidinophenylmethylidenamino, Tautomere davon, und ihre Salze.

Des weiteren bevorzugt sind die Verbindungen der Formel I, worin A eine einfache Bindung oder eine Gruppe NH bedeutet; $X_1$ und $X_2$ unabhängig voneinander CH oder N bedeuten mit der Massgabe, dass mindestens eine der Gruppen $X_1$ und $X_2$ CH bedeutet; $X_3$ und $X_4$ für CH stehen; Y für NH oder auch - falls A eine einfache Bindung bedeutet - für O steht; Z für NH oder S steht; der Rest $R_3$ Wasserstoff oder Niederalkyl bedeutet; $R_1$, $R_2$, $R_4$ und $R_5$ Wasserstoff bedeuten und $R_6$ für Wasserstoff, Niederalkyl, Hydroxy oder Amino steht, Tautomere davon, und ihre Salze.

Als Untergruppen aus einer Gruppe von Verbindungen der Formel I sind jeweils hervorzuheben: (a) Verbindungen der Formel I, worin A eine einfache Bindung bedeutet, (b) Verbindungen der Formel I, worin A eine einfache Bindung bedeutet und Y für NH steht, (c) Verbindungen der Formel I, worin Z für NH steht und $R_5$ und $R_5$ Wasserstoff bedeuten, (d) Verbindungen der Formel I, worin $X_1$ für N steht und $X_2$, $X_3$ und $X_4$ CH bedeuten, (e) Verbindungen der Formel I, worin $X_1$, $X_2$, $X_3$ und $X_4$ CH bedeuten, (f) Verbindungen der Formel I, worin $X_1$ und $X_4$ für N stehen und $X_2$ und $X_3$ CH bedeuten, und (g) Verbindungen der Formel I, worin $X_1$ und $X_2$ für N stehen und $X_3$ und $X_4$ CH bedeuten.

Die Erfindung betrifft vor allem die in den Beispielen beschriebenen spezifischen Verbindungen und Salze davon.

Die Verbindungen der Formel I können in an sich bekannter Weise hergestellt werden, indem man z.B.

a) eine Verbindung der Formel II

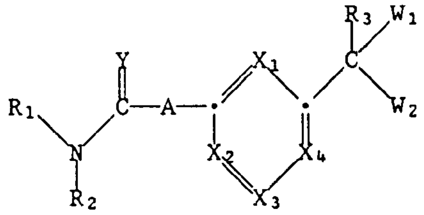

(II)

worin die Gruppe $CW_1W_2$ für Carbonyl, funktionell abgewandeltes Carbonyl oder geschütztes Carbonyl steht, mit einem Amin der Formel III

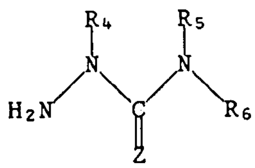

(III)

kondensiert, oder

b) zur Herstellung einer Verbindung der Formel I, worin A eine Gruppe-$NR_7$- bedeutet, eine Verbindung der Formel IV

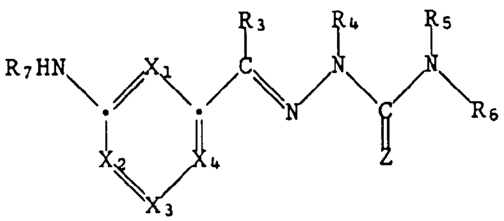

(IV)

mit einem Reagens umsetzt, das geeignet ist, die Gruppe -$NHR_7$ in ein Guanidin der Formel I umzuwandeln, oder

c) zur Herstellung einer Verbindung der Formel I, worin A eine einfache Bindung bedeutet, in einer Verbindung der Formel VI

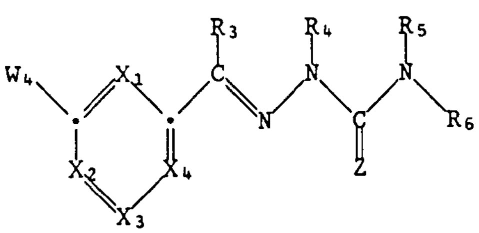

(VI)

worin $W_4$ einen Rest, der in ein Amidin bzw. eine Carbamoylverbindung der Formel I überführt werden kann, bedeutet, den Rest $W_4$ in die Gruppe -C(=Y)$NR_1R_2$ überführt; wobei in den obigen Ausgangsstoffen der Formeln II bis VI die Symbole A, $X_1$, $X_2$, $X_3$, $X_4$, Y, Z und $R_1$-$R_7$ die unter Formel I angegebene Bedeutung haben; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

In der folgenden näheren Beschreibung der Verfahren a)-c) haben die Symbole A, $X_1$, $X_2$, $X_3$, $X_4$, Y, Z und $R_1$-$R_9$ jeweils die unter Formel I angegebene Bedeutung, sofern nichts anderes angegeben ist.

Verfahren a):

Als funktionell abgewandeltes bzw. geschütztes Carbonyl $CW_1W_2$ sind beispielhaft zu nennen: Diniederalkoxymethyl, $C_1$-$C_2$-Alkylendioxymethyl, Dihalogenmethyl, Diniederalkylthiomethyl oder $C_1$-$C_2$-Alkylendithiomethyl.

Die Kondensationsreaktion gemäss Verfahren a) erfolgt unter den an sich bekannten Bedingungen bei der Bildung von Hydrazonen. Sie ist bevorzugt durch Säure katalysiert. In Verbindungen der Formel II sind solche geschützte Carbonylgruppen $CW_1W_2$ geeignet, die unter den Bedingungen der Kondensation in freies Carbonyl übergehen.

Zur Herstellung von Verbindungen der Formel I, worin $R_6$ Amino bedeutet, empfiehlt es sich, die Verbindung der Formel III im Ueberschuss einzusetzen. Verwendet man stöchiometrische Mengen der Verbindung der Formel III, so erhält man normalerweise ein Gemisch bestehend aus einer Verbindung der Formel I, worin $R_6$ Amino bedeutet, und einer anderen Verbindung der Formel I, worin $R_6$ einem Rest T wie oben definiert entspricht. Ausschliesslich die letzteren Verbindungen der Formel I erhält man, wenn man 1 Aequivalent einer Verbindung der Formel II mit 0,5 Aequivalenten einer Verbindung der Formel III umsetzt.

Die Zwischenprodukte der Formel II, worin A eine einfache Bindung und Y NH bedeutet, werden z.B. dadurch erhalten, dass man eine Verbindung der Formel VII

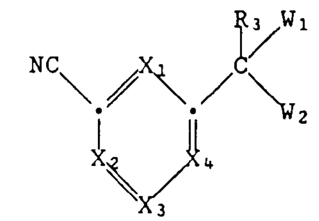

(VII)

worin die Gruppe $CW_1W_2$ wie unter Formel II definiert ist, z.B. mit Ethanol und Salzsäure in z.B. Chloroform oder Diethylether behandelt, wobei das entsprechende Iminoethylester-Hydrochlorid gebildet wird, welches z.B. durch Umsetzung mit Ammoniak oder einem primären oder sekundären Amin der Formel $NHR_1R_2$ und z.B. Methanol in das gewünschte Carboximidamid der Formel II überführt werden kann.

Eine weitere Möglichkeit zur Herstellung der Verbindungen der Formel II besteht darin, eine Verbindung der Formel VII zunächst durch Behandlung mit Schwefelwasserstoff in das entsprechende Thioncarboxamid [-C(=S)-$NH_2$] zu überführen. Letzteres kann auch auf anderem Wege ausgehend vom analogen Carboxamid [-C(=O)-$NH_2$] z.B. durch Umsetzung mit dem Lawesson-Reagens [2,4-Bis-(4-methoxyphenyl)-2,4-dithioxo-1,3,2,4-dithiadiphosphetan] erhalten werden. Die Thioncarboxamide werden z.B. mit Niederalkyliodiden S-alkyliert und damit in Imino-niederalkylthiolester-Hydroiodide [-C(=NH)-S-Alkyl · HI] überführt, welche sich leicht durch Umsetzung mit Ammoniak bzw. Aminen der Formel $NHR_1R_2$ in die gewünschten Carboximidamide der Formel II überführen lassen [vgl. S. Patai (Ed.), The Chemistry of amidines and imidates, Wiley, London etc. 1975, S. 303-304].

Zwischenprodukte der Formel II, worin A die Gruppe -$NR_7$- bedeutet und Y für $NR_6$ steht, sind an sich bekannt oder werden z.B. dadurch hergestellt, dass man eine Verbindung der Formel VIII

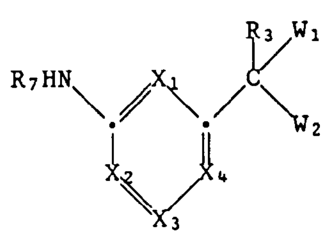

(VIII)

worin die Gruppe $CW_1W_2$ wie unter Formel II definiert ist, mit einem S-Alkylisothiuroniumsalz der Formel IX

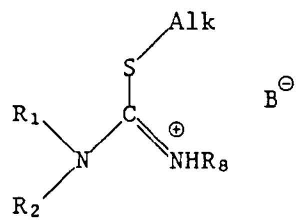

(IX)

worin Alk Niederalkyl bedeutet und $B^{\ominus}$ ein Anion darstellt, umsetzt, wobei die gewünschte Guanylverbindung der Formel II erhalten wird.

Die Herstellung von Verbindungen der Formel VII ist an sich bekannt [vgl. z.B. J.Org.Chem. 44, 2702 (1979)]. Verbindungen der Formel VII, worin $R_3$ für Wasserstoff steht und (a) $X_4$ = N, $X_1$ = $X_2$ = $X_3$ = CH, (b) $X_1$ = $X_2$ = N, $X_3$ = $X_4$ = CH, (c) $X_1$ = $X_4$ = N, $X_2$ = $X_3$ = CH, (d) $X_2$ = $X_4$ = N, $X_1$ = $X_3$ = CH, (e) $X_1$ = $X_3$ = N, $X_2$ = $X_4$ = CH, (f) $X_2$ = $X_3$ = N, $X_1$ = $X_4$ = CH oder (g) $X_3$ = $X_4$ = N, $X_1$ = $X_2$ = CH bedeuten, lassen sich z.B. aus den entsprechenden Cyanomethylverbindungen der Formel X

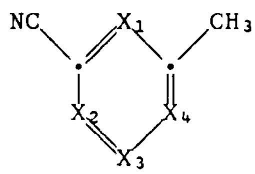

(X)

durch Oxidation erhalten, z.B. nach dem King-Kröhnke-Verfahren, bei dem man die zu oxidierende Methylverbindung der Formel X zunächst mit $J_2$/Pyridin und dann mit p-Nitroso-N,N-dimethylanilin umsetzt und schliesslich mit Salzsäure hydrolysiert (vgl. z.B. Houben-Weyl, Band E3, Thieme Verlag Stuttgart, New York 1983, S. 232).

Die Herstellung von Verbindungen der Formel X ist an sich bekannt. Eine Verbindung der Formel X, worin $X_2$ = $X_3$ = N und $X_1$ = $X_4$ = CH bedeutet, lässt sich z.B. aus 5-Methylpyridazin-3-on durch Umsetzung mit $PCl_5$ oder $POCl_3$ und Reaktion der erhaltenen 3-Chlor-Verbindung mit z.B. Kupfer(I)cyanid in Dimethylformamid bei erhöhter Temperatur erhalten. Ganz analog kann man eine Verbindung der Formel X, worin $X_3$ = $X_4$ = N und $X_1$ = $X_2$ = CH bedeuten, z.B. aus 6-Methylpyridazin-4-on durch Umsetzung mit $PCl_5$ oder $POCl_3$ und Reaktion der erhaltenen 4-Chlorverbindung mit Cu(I)CH in DMF erhalten.

Verbindungen der Formel VII - insbesondere solche, worin $X_1$ und $X_4$ für N stehen und $X_2$ und $X_3$ CH bedeuten - lassen sich z.B. auch auf folgendem Weg herstellen: Ausgehend von 6-Hydroxy-2,4-dimethylpyrimidin wird (a) durch selektive Kondensation der 2-Methylgruppe mit Benzaldehyd, (b) Umwandlung der 6-Hydroxygruppe in 6-Chlor mittels eines Chlorierungsmittels, z.B. $POCl_3$, und (c) selektive Entfernung des 6-Chlorsubstituenten durch Reduktion, z.B. mit $H_2$/Lindlar-Katalysator (5 % Pd auf $CaCO_3$, mit Pb vergiftet), das 4-Methyl-2-(2-phenylethenyl)-pyrimidin erhalten. In letzterem wird die 4-Methylgruppe (d) z.B. durch Behandlung mit metallischem Natrium und n-Butylnitrit in 4-(N-Hydroxyiminomethyl) (Oxim) und letzteres (e) z.B. durch Behandlung mit $POCl_3$ in eine 4-Cyanogruppe überführt. (f) Eine Ozonolyse des erhaltenen 4-Cyano-2-(2-phenylethenyl)-pyrimidins führt schliesslich zur gewünschten Verbindung der Formel VII.

Verbindungen der Formel VII, worin $R_3$ für Niederalkyl steht, lassen sich in an sich bekannter Weise aus entsprechenden Verbindungen der Formel VII herstellen, worin $R_3$ Wasserstoff bedeutet. Z.B. gelingt dies (a) durch Umsetzung mit Cyclohexylamin, Behandlung des entstandenen Azomethins mit Natriumhydrid und einem Diniederalkylsulfoxid und abschliessender saurer Hydrolyse [s. Can.J.Chem. 48, 570 (1970)], (b) durch Ueberführung des Aldehyds ($R_3$ = H) in das entsprechende 1,2-Ethylenacetal oder 1,3-Propylenacetal und Umsetzung desselben mit Niederalkyllithium [s. J.Org.Chem. 30, 226 (1965)] oder (c) durch Umsetzung mit Diazoniederalkan, jeweils gegebenenfalls nach vorherigem Schutz der Cyanofunktion im Molekül durch eine an sich bekannte Cyanoschutzgruppe und abschliessender Abspaltung derselben.

Die Herstellung von Verbindungen der Formel VIII ist an sich bekannt. So können Verbindungen der Formel VIII, und zwar bevorzugt solche, worin $X_1$ und/oder $X_2$ N bedeuten, z.B. durch Umsetzung einer entsprechenden Halogenverbindung der Formel XI

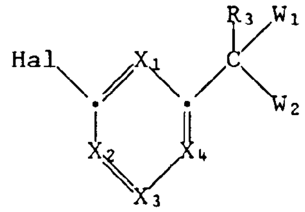

(XI)

worin Hal Halogen bedeutet und die Gruppe $CW_1W_2$ wie unter Formel II definiert ist, mit Ammoniak bzw. einem Amin der Formel $R_7NH_2$ erhalten werden.

Verbindungen der Formel VIII, worin $R_7$ Wasserstoff bedeutet, lassen sich ferner z.B. aus entsprechenden Verbindungen der Formel VII herstellen, indem man zunächst die Cyanogruppe zu Carboxy verseift und anschliessend z.B. einen Curtius-Abbau durchführt, d.h. die Carboxygruppe in das entsprechende Azid überführt und dieses thermisch zersetzt. Besonders einfach gelingt diese Umsetzung, wenn man eine modifizierte Curtius-Reaktion unter Verwendung von $(C_6H_5O)_2P(=O)-N_3$ durchführt [vgl. Tetrahedron 30, 2151-57 (1974)].

Eine weitere Möglichkeit zur Herstellung von Verbindungen der Formel VIII, worin $R_7$ Wasserstoff bedeutet, besteht in der Reduktion entsprechender Nitroverbindungen.

Verbindungen der Formel VIII, worin $R_7$ Niederalkyl bedeutet, lassen sich z.B. aus entsprechenden Verbindungen der Formel VIII, worin $R_7$ für Wasserstoff steht, durch Alkylierung mit Alkylierungsmitteln, z.B. Niederalkyliodiden oder -bromiden, herstellen.

Die Herstellung von Aminoguanidinen, -harnstoffen und -thioharnstoffen der Formel III ist an sich bekannt. Amino(thio)harnstoffe [≙ Semi(thio)carbazide] werden z.B. in analoger Weise hergestellt wie entsprechende (Thio-)Harnstoffe. Dabei werden z.B. anstelle von Aminen die entsprechenden Hydrazine der Formel $H_2N-NHR_4$ eingesetzt und z.B. mit einem Isocyanat der Formel $R_5N=C=O$ oder $R_6N=C=O$, einem Isothiocyanat der Formel $R_5N=C=S$ oder $R_5N=C=S$, einem Carbamoylchlorid der Formel $R_5R_5N-COCl$ oder einem Thioncarbamoylchlorid der Formel $R_5R_5N-CSCl$ umgesetzt. Weiterhin ist z.B. auch die Reaktion eines Hydrazins der Formel $H_2N-NHR_4$ mit einem Acylisothiocyanat, z.B. Acetylisothiocyanat, und nachfolgender saurer Hydrolyse möglich.

Aminoguanidine der Formel III, worin Z für $NR_8$ steht und $R_4$, $R_5$, $R_5$ und $R_8$ wie unter Formel I definiert sind, sind an sich bekannt und lassen sich z.B. aus entsprechenden Aminothioharnstoffen der Formel III herstellen, indem man letztere durch Alkylierung, etwa mit einem Alkyltosylat oder -halogenid, in die entsprechenden S-Alkylisothiuroniumsalze überführt und diese mit einem Amin der Formel $NHR_5R_5$ umsetzt.

<u>Verfahren b):</u>

Als Reagentien, die die Gruppe $-NHR_7$ in einer Verbindung der Formel IV in ein Guanidin der Formel I umwandeln können, sind z.B. Verbindungen der Formel V

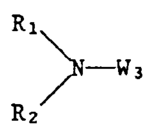

(V)

worin $W_3$ funktionell abgewandeltes Carboxy bedeutet, geeignet.

Die Gruppe $W_3$ in einer Verbindung der Formel V kann bei der Herstellung von Guanidinen der Formel I (Y ≙ $NR_7$) z.B. bedeuten: Cyano oder

$$-\overset{\oplus}{C}(=NHR_7)-S-\text{Niederalkyl}\ B^{\ominus}.$$

Die Bildung von Guanidinen der Formel I bei der Reaktion von Aminen der Formel IV mit gegebenenfalls mono- oder disubstituierten Cyanamiden der Formel V ($W_3$ ≙ CN) bzw. mit gegebenenfalls mono- oder disubstituierten S-Alkylthiuroniumsalzen der Formel V ($W_3$ ≙ $-C(=NHR_7^{\oplus})-S-$Niederalkyl $B^{\ominus}$) ist an sich bekannt.

Die Zwischenprodukte der Formel IV werden z.B. hergestellt, indem man eine Verbindung der Formel VIII, worin die Gruppe $CW_1W_2$ wie unter Formel II definiert ist, mit einem Amin der Formel III kondensiert. Die dabei

ablaufende Reaktion entspricht dem oben beschriebenen Verfahren a). Die Aminogruppe $NHR_7$ im Zwischenprodukt der Formel IV ist dabei gegebenenfalls vor der Reaktion durch eine der in der organischen Chemie üblicherweise verwendeten $NH_2$-Schutzgruppen zu schützen. Nach erfolgter Kondensation lässt sich dann die Schutzgruppe in an sich bekannter Weise abspalten und man erhält ein Zwischenprodukt der Formel IV.

Verfahren c):

In den Zwischenprodukten der Formel VI bedeutet $W_4$ z.B. freies oder funktionell abgewandeltes Carboxy, insbesondere Halogencarbonyl, Cyan, Imino-niederalkoxycarbonyl oder Imino-niederalkylthiolcarbonyl.

Die Gruppe $W_4$ in einer Verbindung der Formel VI kann bei der Herstellung von Amidinen der Formel I (Y $\triangleq$ $NR_7$) z.B. bedeuten: ein Säureadditionssalz eines Imino-niederalkylesters ($\triangleq$ Iminoniederalkylether) oder Imino-niederalkylthiolesters, z.B. $-C(=NH)-OC_2H_5 \cdot HCl$ bzw. $-C(=NH)-SC_2H_5 \cdot HI$, oder Cyano.

Durch die Umsetzung eines Imino-niederalkylesters der Formel VI (als Salz) mit Ammoniak oder primären bzw. sekundären Aminen erhält man die unsubstituierten bzw. mono- oder disubstituierten Amidine der Formel I. Cyanoverbindungen der Formel VI können z.B. durch Umsetzung mit einem Alkalimetallamid, z.B. $KNH_2$, oder durch Reaktion mit einem primären oder sekundären (Di-)niederalkylammoniumhalogenid, z.B.

$$\overset{\oplus}{N}H_3CH_3\ Cl^{\ominus},$$

in ein gegebenenfalls mono- oder disubstituiertes Amidin der Formel I überführt werden.

Setzt man einen Imino-niederalkylester der Formel VI mit einem $\alpha,\omega$-Diaminoalkan, z.B. 1,2-Diaminoethan oder 1,3-Diaminopropan, um, so erhält man eine Verbindung der Formel I, worin die Reste $R_1$ und $R_8$ zusammen Alkylen bedeuten.

Die Gruppe $W_4$ in einer Verbindung der Formel VI kann bei der Herstellung von Carbamoylverbindungen der Formel I (Y $\triangleq$ O) z.B. bedeuten: Carboxy, Halocarbonyl (z.B. -COCl), Niederalkoxycarbonyl oder Cyano. Die Bildung von gegebenenfalls mono- oder disubstituierten Carbamoylverbindungen der Formel I aus entsprechenden Zwischenprodukten der Formel VI, worin $W_4$ Carboxy, Halocarbonyl oder Niederalkoxycarbonyl bedeutet, durch Umsetzung mit Ammoniak bzw. primären oder sekundären Aminen ist an sich bekannt. Zwischenprodukte der Formel VI, worin $W_4$ Cyano bedeutet, können z.B. durch partielle Hydrolyse, im Sinne einer Graf-Ritter-Reaktion, oder über Carbonsäureesterimid-Salze in gegebenenfalls mono- oder disubstituierte Carbamoylverbindungen der Formel I überführt werden. Die Bedingungen bei der Hydrolyse der Cyano-Zwischenprodukte können so gewählt werden, dass die Reaktion auf der Stufe des Amids abgebrochen wird. Zu diesem Zweck ist insbesondere die Hydrolyse mit Säuren, wobei z.B. 80 %ige Schwefelsäure (unter Erwärmen), Polyphosphorsäure (bei 110-150°C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen.

Mit Hilfe der Graf-Ritter-Reaktion gelingt auch die Herstellung N-substituierter Amide aus Nitrilen der Formel VI. Hierzu setzt man die Nitrile in Gegenwart einer starken Säure, vornehmlich 85-90 %iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Ethanol.

Die Carbonsäureesterimide erhält man z.B. durch säurekatalysierte Anlagerung von Alkoholen an die Nitrile der Formel VI. Aus den Esterimiden erhält man die Amide im Sinne einer Pinner-Spaltung durch thermischen Zerfall der Esterimid-Salze bei Temperaturen oberhalb von etwa 80°C.

Verbindungen der Formel VI, worin $W_4$ Cyano bedeutet, können z.B. dadurch hergestellt werden, dass man eine Verbindung der Formel VII mit einer Verbindung der Formel III gemäss dem Verfahren a) umsetzt. Aus Verbindungen der Formel VI, worin $W_4$ Cyano bedeutet, lassen sich die anderen Verbindungen der Formel VI, worin $W_4$ freies oder anderweitig funktionell abgewandeltes Carboxy bedeutet, in an sich bekannter Weise oder wie oben beschrieben herstellen.

Verbindungen der Formel I können in andere Verbindungen der Formel I überführt werden.

Zum Beispiel erhält man bei der Umsetzung einer Verbindung der Formel I, worin $R_8$ Amino bedeutet, mit einer Verbindung der Formel II eine Verbindung der Formel I, worin $R_8$ einen Rest T wie oben definiert bedeutet. Auf diese Weise lassen sich auch entsprechende Verbindungen der Formel I herstellen, die unsymmetrisch in bezug auf ihre Gruppierung $-N(R_4)-C(=Z)-N(R_8)-$ sind.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat.

Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 % vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,1 g bis etwa 10 g, vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Grad Celsius angegeben.

Beispiel 1: 3-Formylbenzamidin-2′-amidinohydrazon-dihydrochlorid

Rohes 3-Formylbenzamidin-hydrochlorid wird in 200 ml 96-proz. Ethanol gelöst und zu einer Mischung von 12,3 g (0,1 Mol) Aminoguanidin-sulfat, 60 ml Wasser und einigen Tropfen konzentrierter Schwefelsäure gegeben und das Ganze während 15 min unter Rückfluss gekocht. Beim Abkühlen kristallisieren 20 g Produkt als Sulfat aus. Dieses wird zu einer Lösung von 6,3 g (0,15 ,Mol) NaOH in 400 ml Ethanol gegeben und 2,5 h auf 70° erhitzt. Nach dem Abkühlen wird ungelöstes Material abfiltriert und das Filtrat eingedampft. Der Rückstand wird in wenig Ethanol gelöst und mit 10-proz. ethanolischer Salzsäure sauer gestellt. Nach dem Filtrieren durch Kieselgur Hyflo Super Cel®, Fluka) wird im Vakuum bis zur einsetzenden Kristallisation eingeengt. Man erhält so die Titelverbindung, Smp. 206-207°.

Die Ausgangsverbindungen werden wie folgt hergestellt:

(a) Ethyl-3-formylbenzimidat-hydrochlorid

Eine Lösung von 86,7 g (0,662 Mol) 3-Formylbenzonitril in 530 ml abs. Aether wird mit 59,7 ml (1,025 Mol) abs. Ethanol versetzt und auf 0° abgekühlt. Die Reaktionslösung wird mit trockenem Salzsäure-Gas gesättigt und anschliessend sechs Tage bei 0° stehengelassen. Nach dem Abfiltrieren eines feinen Niederschlags wird die Reaktionslösung mit 1ℓ Ether versetzt. Dabei kristallisiert die Titelverbindung aus, Smp. 126-128° (unter Aufschäumen).

(b) 3-Formylbenzamidin-hydrochlorid

21,3 g (0.1 Mol) des Iminoethers aus Beispiel 1a werden mit 250 ml abs. Ethanol und 250 ml gesättigter ethanolischer Ammoniaklösung versetzt und während 3 h auf 70° erhitzt. Nach dem Abkühlen wird das Ethanol abgedampft und der Rückstand, der der Titelverbindung in roher Form entspricht, weiter umgesetzt.

Beispiel 2: 3-Formylbenzamid-2′-amidinohydrazon-halbsulfat

Zu einer Lösung von 5,0 g (0,033 Mol) 3-Formylbenzamid in 70 ml Ethanol wird eine Lösung von 4,05 g

(0,033 Mol) Aminoguanidin-sulfat in 20 ml Wasser gegeben. Nach Zugabe von 3 Tropfen konz. Schwefelsäure wird das Reaktionsgemisch während 2 h unter Rückfluss gekocht.

Beim Abkühlen kristallisiert das Produkt aus. Dieses wird abfiltriert, mit Ethanol verkocht, abgekühlt, wieder filtriert und schliesslich aus Wasser umkristallisiert, Smp. 270° (Zers.).

Beispiel 3: 3-Acetylbenzamidin-2′-amidinohydrazon-dihydrochlorid

11,4 g (0,05 Mol) des Iminoethers aus Beispiel 3a, 200 ml Ethanol und 125 ml gesättigte ethanolische Ammoniaklösung werden während 6 h unter Rückfluss gekocht. Nach dem Abkühlen wird das Reaktionsgemisch eingedampft, der Rückstand in 4 N Salzsäure gelöst, mit Ether gewaschen und zur Trockne eingedampft. Das erhaltene rohe 3-Acetylbenzamidin-hydrochlorid wird in 75 ml Methanol gelöst und mit einer Lösung von Aminoguanidin-hydrochlorid [hergestellt aus 7,5 g (0,055 Mol) Aminoguanidin-hydrogencarbonat in 40 ml Wasser und 54 ml 2 N Salzsäure] versetzt. Dieses Gemisch wird anschliessend während 1,5 h auf 70° erhitzt und danach eingedampft. Der Rückstand wird aus Ethanol umkristallisiert und man erhält die Titelverbindung, Smp. 185°.

Die Ausgangsverbindungen werden wie folgt hergestellt:

(a) Ethyl-3-acetylbenzimidat-hydrochlorid

Eine Lösung von 7,25 g (0,05 Mol) 3-Acetylbenzonitril in 150 ml Ether und 4,5 ml Ethanol wird bei 0° mit trockenem Salzsäuregas gesättigt und anschliessend 2 Tage bei 0° stehengelassen. Das auskristallisierte Produkt wird abfiltriert und aus Ethanol/Ether umkristallisiert; Smp. 110° (Zers.).

Beispiel 4: 1-Hydroxy-3-(3′-amidinobenzyliden-amino)-guanidin-dihydrochlorid (≙ 3-Formylbenzamidin-2′-(N-hydroxyamidino)-hydrazon-dihydrochlorid)

Eine Lösung von 6,9 g (0,028 Mol) rohem 3-Formyl-benzamidin-hydrochlorid (hergestellt wie unter Beispiel 1b beschrieben) in 28 ml Methanol wird mit einer Lösung von 7,3 g (0,028 Mol) 1-Amino-3-Hydroxyguanidin-toluolsulfonat in 14 ml Wasser und 14,5 ml 2 N Salzsäure versetzt und 1 h auf 70° erhitzt. Das Reaktionsgemisch wird abgekühlt, eingedampft und das Rohprodukt aus Wasser/Ethanol umkristallisiert; Smp. 248-250°.

Beispiel 5: N,N′-Dimethylamidinobenzaldehyd-2′-amidinohydrazon-dihydrochlorid

Zu einer Lösung von 6,71 g (0,049 Mol) Aminoguanidin-hydrogencarbonat in 37 ml Wasser und 53,4 ml 2 N Salzsäure wird eine Lösung von 8,11 g (~0,05 Mol) N,N′-Dimethylamidinobenzaldehyd in 80 ml Methanol zugetropft. Das Reaktionsgemisch wird 30 min unter Rückfluss gekocht und anschliessend 24 h bei Raumtemperatur stehengelassen. Nach dem Eindampfen wird das Rohprodukt mittels Chromatographie an Amberlite® XAD 1180 (Wasser als Eluiermittel) gereinigt und schliesslich aus 2 N Salzsäure/Ethanol umkristallisiert; Smp. 103-105°.

Die Ausgangsverbindungen werden wie folgt hergestellt:

(a) N,N′-Dimethylamidinobenzaldehyd

10,7 g (0,05 Mol) Iminoether aus Beispiel 1a werden in 125 ml abs. Ethanol suspendiert und langsam mit 125 ml einer 33-proz. Lösung von Methylamin in Ethanol (Hersteller: Fluka) versetzt. Nach 3 h bei 70° wird das klare, hellbraune Reaktionsgemisch eingedampft, in 50 ml 2 N Salzsäure gelöst und mit Ether gewaschen. Die wässrige Phase wird eingedampft und der Rückstand, der der Titelverbindung in roher Form entspricht, direkt weiterverarbeitet.

Beispiel 6: 3-Formylbenzamidin-thiosemicarbazon-hydrochlorid

Zu einer Lösung von 0,91 g (0,01 Mol) Thiosemicarbazid in 5 ml Wasser und 10 ml 2 N Salzsäure wird eine Lösung von 1,84 g (~0,01 Mol) rohes 3-Formylbenzamidin-hydrochlorid (hergestellt analog Beispiel 1b) in 10 ml Methanol getropft. Dieses Gemisch wird 1 h unter Rückfluss erhitzt, abgekühlt, filtriert und zur Trockne eingedampft. Der Rückstand wird aus Methanol umkristallisiert; Smp. 210° (Zers.). Das Produkt enthält 0,5 Mol Wasser.

Beispiel 7: 3-Formyl-N,N-dimethylbenzamidin-2′-amidinohydrazon-dihydrochlorid-hydrat

Zu einer Lösung von 6,6 g (0,049 Mol) Aminoguanidin-hydrogencarbonat in 35 ml Wasser und 51 ml 2 N Salzsäure wird eine Lösung von 11,7 g (~0,05 Mol) rohem 3-Formyl-N,N-dimethylbenzamidin-hydrochlorid in 50 ml Methanol getropft. Das Reaktionsgemisch wird 30 min unter Rückfluss erhitzt und anschliessend eingedampft. Der Rückstand wird an Amberlite® XAD 1180 (mit Wasser als Eluiermittel) chromatographiert. Lyophilisat-"Smp." 80-82°.

Die Ausgangsverbindungen werden wie folgt hergestellt:

(a) 3-Formyl-N,N-dimethylbenzamidin-hydrochlorid

10,7 g (0,05 Mol) des Iminoethers aus Beispiel 1a werden in 125 ml abs. Ethanol aufgeschlemmt und tropfenweise mit 125 ml einer 33-proz. Dimethylamin-Lösung in Ethanol versetzt. Nach einer Reaktionszeit von 3 h bei 70° wird eingedampft, der Rückstand in 50 ml 2 N Salzsäure gelöst, mit Ether gewaschen und erneut zur Trockne eingedampft. Das resultierende Oel, das der Titelverbindung in roher Form entspricht, wird ohne Reinigung weiter umgesetzt.

Beispiel 8: 2-Amidino-6-formylpyridin-2′-amidinohydrazon-dihydrochlorid und 2-Carbamoyl-6-formylpyridin-2′-amidinohydrazon-dihydrochlorid

12,5 g (0,09 Mol) Aminoguanidin-hydrogencarbonat werden in 75 ml Wasser und 100 ml 2 N Salzsäure gelöst und bei Raumtemperatur mit einer Lösung von ~33 g (~0,1 Mol) rohem 2-Amidino-6-formylpyridin-hydrochlorid in 100 ml Methanol versetzt. Das Reaktionsgemisch wird 1,5 h unter Rückfluss erhitzt, abgekühlt und eingedampft. Der Rückstand wird in wenig heissem 90-proz. Ethanol gelöst und langsam abgekühlt. Dabei kristallisiert das 2-Carbamoyl-6-formylpyridin-2′-amidinohydrazon-dihydrochlorid, Smp. 190°, aus. Es wird abfiltriert, die Mutterlauge eingedampft und der Rückstand an Amberlite® XAD 1180 chromatographiert (Wasser als Eluiermittel). Das als Hauptprodukt erhaltene 2-Amidino-6-formylpyridin-2′-amidinohydrazon-dihydrochlorid wird schliesslich aus Ethanol umkristallisiert; Smp. 160°.

Die Ausgangsverbindungen werden wie folgt hergestellt:

(a) 2-Amidino-6-formylpyridin-hydrochlorid

2-Cyano-6-formylpyridin (16,5 g ≙ 0,125 Mol) wird in 150 ml trockenem Ether und 150 ml abs. Ethanol gelöst und bei 0° mit trockenem Salzsäuregas gesättigt und 44 h bei 0° stehengelassen. Die rotbraune Reaktionslösung wird zur Trockne eingeengt. Zum Entfernen überschüssiger Salzsäure wird der Rückstand nochmals in absolutem Ethanol gelöst, erneut eingedampft und zuletzt am Hochvakuum getrocknet. Der resultierende rohe Iminoether wird in 100 ml abs. Ethanol gelöst und mit einer gesättigten ethanolischen Ammoniaklösung (100 ml) versetzt. Das Reaktionsgemisch wird 3 h unter Rückfluss erhitzt, abgekühlt, klarfiltriert und das Filtrat eingedampft. Die Lösung des Rückstandes in 100 ml 2 N Salzsäure wird mit Ether gewaschen bis zur Trockne eingedampft und am Hochvakuum getrocknet. Man erhält die Titelverbindung in roher Form, welche direkt weiterverarbeitet wird.

Beispiel 9: 2-Amidino-4-formylpyridin-2′-amidinohydrazon-dihydrochlorid

11,2 g (0,08 Mol) Aminoguanidin-hydrogencarbonat werden in 90 ml Wasser und 104 ml 2 N Salzsäure gelöst und mit einer Lösung von 52 g (~0,11 Mol) rohem 2-Amidino-4-formylpyridin-diethylacetal-hydrochlorid in 180 ml Ethanol versetzt. Das resultierende Gemisch wird 1,5 h unter Rückfluss erhitzt, abgekühlt und eingedampft. Der Rückstand wird an Amberlite® XAD 1180 mit Wasser als Eluiermittel chromatographiert. Die Fraktionen, welche nach Dünnschichtchromatographie das gewünschte Produkt enthalten, werden eingedampft und der Rückstand aus Ethanol/Ether umkristallisiert; Smp. 290° (Zers.).

Die Ausgangsverbindungen werden wie folgt hergestellt:

(a) Ethyl-4-formyl-2-pyridyl-imidat-diethylacetal-hydrochlorid

27 g (0,125 Mol) 2-Cyano-4-formylpyridin-diethylacetal werden in 250 ml trockenem Ether und 11 ,25 ml abs. Ethanol gelöst. Diese Lösung wird bei 0° mit trockenem Salzsäuregas gesättigt (ca. 50 g) und 1 h bei 0° gerührt. Das ausgefallene Produkt wird abfiltriert, mit kaltem Ether gewaschen und getrocknet; Smp. 95-97°.

(b) 2-Amidino-4-formylpyridin-diethylacetal-hydrochlorid

37,0 g (0,128 Mol) des Iminoethers aus Beispiel 9a werden in 277 ml abs. Ethanol gelöst und bei Raumtemperatur mit 470 ml einer gesättigten ethanolischen Ammoniaklösung versetzt. Nach 2 h bei 70° (Rückfluss) wird abgekühlt, eingedampft, der Rückstand in 2 N Salzsäure gelöst, mit Ether gewaschen und zuletzt nochmals zur Trockne eingedampft. Das noch leicht feuchte Rohprodukt wird ohne weitere Reinigung weiterverarbeitet.

Beispiel 10: 3-Amino-1-(3′-Amidinobenzyliden-amino)-guanidin-dihydrochlorid und 1,3-Bis(3′-Amidinobenzyliden-amino)-guanidin-trihydrochlorid

1,25 g (0,01 Mol) 1,3-Diaminoguanidin-hydrochlorid werden in 10 ml Wasser gelöst und bei Raumtemperatur mit einer Lösung von 1,84 g (~0,01 Mol) rohem 3-Formylbenzamidin-hydrochlorid (Beispiel 1b) in 10 ml Methanol versetzt. Das Gemisch wird 16 h bei Raumtemperatur gerührt und danach eingedampft. Der Rückstand wird in Ethanol aufgenommen, durch Filtration von ungelöstem Material befreit und erneut eingedampft. Das resultierende Oel wird an Amberlite® XAD 1180 mit Wasser chromatographiert. Die Fraktionen 10-13 entsprechen dem 3-Amino-1-(3′-Amidinobenzyliden-amino)-guanidin-dihydrochlorid (Zers. bei ~160°) und die Fraktionen 15-20 entsprechen dem 1,3-Bis(3′-Amidinobenzyliden-amino)-guanidin-trihydrochlorid-dihydrat (Zers. bei ~180°).

Beispiel 11: 3-Formyl-N-n-butylbenzamidin-2′-amidinohydrazon-dihydrochlorid

1,22 g (0,0099 Mol) Aminoguanidin-hydrogencarbonat werden in 10 ml Wasser und 10 ml 2 N Salzsäure gelöst und bei Raumtemperatur mit einer Lösung von ca. 2,4 g (~0,01 Mol) rohem 3-Formyl-N-n-butylbenzamidin-hydrochlorid in 15 ml Ethanol versetzt. Das Gemisch wird abgekühlt, zur Trockne eingedampft und an einer Säule mit Amberlite® XAD 1180 chromatographiert (Wasser als Eluiermittel). Nach Umkristallisation aus Ethanol/Ether erhält man die Titelverbindung, Smp. 230°.

Die Ausgangsverbindungen werden wie folgt hergestellt:

(a) 3-Formyl-N-n-butylbenzamidin-hydrochlorid

2,1 g (0,01 Mol) Ethyl-3-formylbenzimidat-hydrochlorid (Beispiel 1a) in 15 ml abs. Ethanol werden unter Rühren bei Raumtemperatur mit 2,0 ml (0,02 Mol) n-Butylamin versetzt. Das Reaktionsgemisch wird 3 h unter Rückfluss erhitzt, abgekühlt und direkt weiter umgesetzt.

Beispiel 12: 3-Formylbenzamidin-2′-(N-n-butylamidino)-hydrazon-dihydrochlorid

Eine Lösung von ca. 1,8 g (0,01 Mol) 3-Formylbenzamidin-hydrochlorid (Beispiel 1b) wird bei Raumtemperatur mit einer Lösung von 1,66 g (0,01 Mol) 1-Amino-3-n-butylguanidin-hydrochlorid in 30 ml 50-proz. Ethanol versetzt und 2 h auf ca. 70° erwärmt (Rückfluss). Die klare Reaktionslösung wird abgekühlt, eingedampft und der Rückstand an einer Säule Amberlite® XAD 1180 (Wasser als Eluiermittel) chromatographiert. Die Fraktionen 14-30 ergeben, nach Umkristallisation aus Ethanol/Ether, die Titelverbindung mit einem Zersetzungspunkt von 140°.

Beispiel 13: N-(3-Acetylphenyl-2′-amidinohydrazon)-guanidin-dihydrochlorid

Eine Suspension von 14,0 g 3-Aminoacetophenon-2′-amidinohydrazon-hydrochlorid in 360 ml Ethanol und 102,8 ml 0.6 N ethanolischer Salzsäure wird mit 15,4 g Cyanamid ($H_2N$-CN) versetzt und 65 h am Rückfluss gekocht. Das Reaktionsgemisch wird heiss filtriert, die erhaltenen Kristalle werden mit wenig Ethanol nachgewaschen und dann im Vakuum bei 40° getrocknet. Man erhält so die Titelverbindung, Smp. >250°.

Beispiel 14: 3-Formyl-N-n-butylbenzamidin-2′-(N-n-butylamidino)-hydrazon-dihydrochlorid

1,7 g (0,01 Mol) 1-Amino-3-n-butylguanidin-hydrochlorid werden in 30 ml 50-proz. Ethanol gelöst und mit einer Lösung von ca. 2,4 g (0,01 Mol) rohem 3-Formyl-N-n-butylbenzamidin-hydrochlorid (Beispiel 11a) in 20 ml Ethanol versetzt. Das Reaktionsgemisch wird anschliessend 1 h unter Rückfluss erhitzt, abgekühlt, eingedampft und an Amberlite® XAD 1180 chromatographiert (Wasser als Eluiermittel). Die das Produkt enthaltenden Fraktionen werden eingedampft und der Rückstand aus Ethanol/Ether unter Zusatz von ethanolischer

Salzsäure umkristallisiert. Man erhält Kristalle, die sehr hygroskopisch sind; $^1$H-NMR: δ = 0,8-1,0 (m, 6H); 1,20-1,75 (m, 8H); 3,46 (q, J = 6 Hz, 4H). MS: m/e 317 (M + 1), 316 ($M^+$), 244, 202, 176 (100 %).

Beispiel 15: N-(3-Acetylphenyl-2'-amidinohydrazon)-guanidin-dihydrochlorid

Eine Lösung von 1,36 g Aminoguanidin-hydrogencarbonat in 5 ml 4,0 N Salzsäure wird mit einer Lösung von 1,59 g 3-Guanidinoacetophenon [J. Med. Chem. 10, 1123 (1967)] in 10 ml Methanol versetzt und 22 h am Rückfluss gekocht. Das Reaktionsgemisch wird zur Trockne eingedampft und der Rückstand aus 50 ml Ethanol umkristallisiert. Man erhält so die Titelverbindung, Smp. >250°.

Beispiel 16: N-(3-Guanidinobenzylidenamino)-guanidin-dihydrochlorid

Eine Lösung von 2,5 g (10 mMol) 3-Aminobenzylidenamino-guanidin-dihydrochlorid [s. Chem. Abstr. 57, 9826a (1957)] in 90 ml abs. Ethanol wird mit 1,5 g Cyanamid und 12,5 ml 0,95 N ethanolischer Salzsäure versetzt und 89 h am Rückfluss gekocht. Anschliessend wird das Reaktionsgemisch abgekühlt und auf die Hälfte eingeengt. Das auskristallisierte Produkt wird abgesaugt, mit wenig Ethanol gewaschen und getrocknet. Man erhält so die Titelverbindung, Smp. >250°.

Beispiel 17: 4-Amidino-2-formylpyrimidin-2'-amidinohydrazon-dihydrochlorid

100 mg (0,41 mMol) 4-Cyano-2-formylpyrimidin-2'-amidinohydrazon-hydrochlorid werden in 2 ml abs. Methanol gelöst, mit 100 mg Molekularsieb 3 Å versetzt und bei Raumtemperatur unter Argon während 1 h gerührt. Danach gibt man 91 µl einer ca. 5,4 M Lösung von Natriummethanolat in Methanol hinzu und rührt während 2 h bei 50°, dann weitere 3 h bei Raumtemperatur. Nach erfolgtem Umsatz zum Iminoester (HPLC-Kontrolle) gibt man zum Reaktionsgemisch 24,1 mg Ammoniumchlorid (trocken) und rührt während 21 h bei Raumtemperatur. Nach Zugabe einer zweiten Portion Ammoniumchlorid (11 mg) rührt man noch 3 h bei 50°, nutscht über Hyflo Super Cel® (Kieselgur) ab und destilliert das Lösungsmittel unter Vakuum ab. Der Rückstand wird in wenig Wasser gelöst und bei 0° mit 1,64 ml 0,1 N Salzsäure versetzt. Eine "reverse phase"-Chromatographie an Opti-UP-$C_{12}$ Kieselgel (Antecgel-Dodecyltrichlorsilan, Fa. Antec) in Wasser führt nach Verdampfung des Wassers zur Titelverbindung; $^1$H-NMR (DMSO-$d_6$): δ = 9,30 (d,1H); 8,46 (d,1H); 8,35 (s,1H).

Die Ausgangsverbindung wird wie folgt hergestellt:

(a) 6-Hydroxy-4-methyl-2-(2-phenylethenyl)-pyrimidin [vgl. Chem. Pharm. Bull. 13, 1183 (1965)]

60 g (0,483 Mol) 6-Hydroxy-2,4-dimethylpyrimidin [s. Tetrahedron 35, 2087 (1979)] in 180 ml Acetanhydrid werden mit 48,8 ml frisch destilliertem Benzaldehyd versetzt und unter Argon während 23 h auf 130° erwärmt. Man giesst das erkaltete Reaktionsgemisch in 2 l eiskalten Ether, rührt während 1 h unter Eiskühlung nach und nutscht den erhaltenen Niederschlag ab. Die gelbbraunen Kristalle werden mit Ether gewaschen und aus Methanol umkristallisiert, Smp. 217-219°.

(b) 6-Chlor-4-methyl-2-(2-phenylethenyl)-pyrimidin

212 mg (1 mMol) 6-Hydroxy-4-methyl-2-(2-phenylethenyl)-pyrimidin in 1 ml Phosphoroxychlorid werden unter Stickstoff während 35 min am Rückfluss gekocht. Man giesst das erkaltete Reaktionsgemisch auf Eis, neutralisiert die wässrige Lösung mit Natronlauge und extrahiert mehrmals mit Ether. Die Etherextrakte werden mit Wasser und gesättigter Kochsalzlösung gewaschen, über $CaCl_2$ getrocknet, abgenutscht und eingeengt. Die Reinigung erfolgt durch Sublimation im Kugelrohrofen bei 150° Ofentemperatur und unter Vakuum (0,1 mm Hg ≙ 0,13 mbar). Man erhält farblose Kristalle vom Smp. 71-73°.

(c) 4-Methyl-2-(2-phenylethenyl)-pyrimidin

13,65 g (59,17 mMol) 6-Chlor-4-methyl-2-(2-phenylethenyl)-pyrimidin in 220 ml abs. Ethanol werden mit 9,02 ml Triethylamin und 1,365 g Lindlar-Katalysator versetzt und bei Normaldruck und Raumtemperatur hydriert. Nach Aufnahme von 1 Aequivalent Wasserstoff wird die Hydrierung abgebrochen (nach etwa 7,5 h), das Reaktionsgemisch über Hyflo Super Cel® abgenutscht und am Rotationsverdampfer eingeengt. Durch zweimalige Chromatographie an Kieselgel, Laufmittel Chloroform bzw. n-Hexan/Essigester 5 - 20 %, erhält man die reine Titelverbindung; Smp. 65-67°.

(d) 4-N-Hydroxyiminomethyl-2-(2-phenylethenyl)-pyrimidin

In einem mit Aceton/Trockeneis gekühlten 4-Halskolben mit Magnetrührer, Gaseinleitungsrohr, Thermometer und einem mit Trockeneis gefüllten Kühlfinger werden unter Stickstoff 416 ml flüssiger Ammoniak vorgelegt. Zu diesem gibt man unter Stickstoff und unter Rühren 2,91 g (126,7 mMol) in kleine Stücke geschnittenes, metallisches Natrium gefolgt von 100 mg Eisen(III)chlorid (wasserfrei). Man lässt das Gemisch während 20 min unter Rückfluss kochen (Kältebad -20°). Man erhält eine schwarzblaue Lösung, die innerhalb von 10 min mit 8,16 g (41,16 mMol) pulverisiertem 4-Methyl-2-(2-phenylethenyl)-pyrimidin (in 3 Portionen) versetzt wird. Man lässt das Reaktionsgemisch weitere 60 min unter Rückfluss kochen und tropft dann innerhalb von 10 min 9,7 ml (2 Aequivalente) n-Butylnitrit hinzu. Man erhält eine rote Lösung, die man weitere 60 min unter Rückfluss kochen lässt. Danach gibt man zum Reaktionsgemisch 6,93 g (129 mMol) Ammoniumchlorid (portionenweise), entfernt das Kältebad und lässt den Ammoniak bei Raumtemperatur abdampfen. Der feste, graue Rückstand wird mit Aceton aufgekocht, abfiltriert und die Lösung eingeengt. Das Rohprodukt wird durch Chromatographie an Kieselgel, Laufmittel Methylenchlorid/Aceton 5 - 10 %, gereinigt. Die erhaltene Titelverbindung ist ein syn-anti-Gemisch der beiden Oxime. $^1$H-NMR (DMSO-$d_6$): δ = 12,65 und 12,29 (bs, 1H); 8,94/d und 8,80/d (1H); 8,15/d und 7,61/d (1H); 8,09/s und 7,57/s (1H); 7,97/d und 7,96/d (1H); 7,31/d und 7,29/d (1H).

(e) 4-Cyano-2-(2-phenylethenyl)-pyrimidin

425 mg (1,88 mMol) 4-N-Hydroxyiminomethyl-2-(2-phenylethenyl)-pyrimidin (syn-anti-Gemisch) in 4,25 ml $POCl_3$ werden unter Stickstoff während 1 h auf 120° erhitzt. Die auf ca. 70° abgekühlte Lösung giesst man auf ein Gemisch bestehend aus 50 g Eis und 22 ml 28 % wässrige Ammoniaklösung, sättigt mit festem $K_2CO_3$ und extrahiert dreimal mit Essigester. Die organische Phase wird neutral gewaschen, über $Na_2SO_4$ getrocknet und eingeengt. Zur Reinigung kristallisiert man aus Chloroform/Ether um; Smp. 147-150° (farblose Kristalle).

(f) 4-Cyano-2-formylpyrimidin

2,238 g (10,81 mMol) 4-Cyano-2-(2-phenylethenyl)-pyrimidin in 150 ml abs. Methanol und 75 ml abs. Methylenchlorid werden auf -70°C abgekühlt. Es wird solange Ozon durchgeleitet, bis eine blaue Lösung entsteht (ca. 0,5 h). Man leitet Stickstoff durch die Lösung, bis das überschüssige Ozon entfernt ist (ca. 0,75 h) und versetzt dann das Reaktionsgemisch mit 3,5 ml Dimethylsulfid. Man lässt die Lösung auf Raumtemperatur erwärmen und engt am Rotationsverdampfer ein. Das Rohprodukt wird an Kieselgel (Laufmittel: n-Hexan/Essigester 1:3) gereinigt. $^1$H-NMR ($CDCl_3$): δ = 10,11 (s,1H); 9,23 (d,1H); 7,82 (d,1H).

(g) 4-Cyano-2-formylpyrimidin-2′-amidinohydrazon-hydrochlorid

207 mg (1,49 mMol) Aminoguanidin-hydrogencarbonat in 1,14 ml Wasser werden mit 1,65 ml 2 N HCl versetzt gefolgt von 200 mg (1,5 mMol) 4-Cyano-2-formylpyrimidin sowie 1,5 ml Methanol. Man erwärmt das Reaktionsgemisch auf 70°, wobei eine klare Lösung entsteht. Nach 45 min bei dieser Temperatur wird das Reaktionsgemisch weitgehend eingeengt und der entstandene Niederschlag abgenutscht. Man wäscht mit wenig kaltem Wasser und kaltem Ethanol nach und trocknet die Titelverbindung bei 40° am Hochvakuum. $^1$H-NMR (DMSO-$d_6$): δ = 12,65/bs und 7,97/bs (NH; tauschen mit $D_2O$ aus), 9,23 (d,1H); 8,30 (s,1H); 8,16 (s,1H).

Beispiel 18: 4-(N-Methylamidino)-2-formylpyrimidin-2′-amidinohydrazon-dihydrochlorid

Ausgehend von 4-Cyano-2-formylpyrimidin-2′-amidinohydrazon-hydrochlorid (Beispiel 17g) und Methylamin-hydrochlorid erhält man analog Beispiel 17 die Titelverbindung; $^1$H-NMR (DMSO-$d_6$): δ = 9,30 (d,1H); 8,62 (d,1H); 8,38 (s,1H); 3,15 (d,1H).

Beispiel 19: 4-(N-Ethylamidino)-2-formylpyrimidin-2′-amidinohydrazon-dihydrochlorid

200 mg (0,819 mMol) 4-Cyano-2-formylpyrimidin-2′-amidinohydrazon-hydrochlorid (Beispiel 17g) werden unter Argon in 8 ml abs. Methanol vorgelegt und mit 0,3 ml einer 5,4 M methanolischen Natriummethanolat-Lösung versetzt. Nach 6 h Rühren bei 50° kühlt man das Reaktionsgemisch auf Raumtemperatur ab und versetzt es mit 86,9 mg (1,06 mMol) Ethylamin-hydrochlorid. Man rührt über Nacht bei Raumtemperatur, versetzt das Reaktionsgemisch mit weiteren 20 mg (0,24 mMol) Ethylamin-hydrochlorid und rührt weitere 22 h bei Raumtemperatur. Danach erwärmt man noch 1 h auf 50°, kühlt die Lösung ab und engt das Gemisch weitgehend ein. Der Rückstand wird in Wasser aufgenommen, und es wird durch Zugabe von 1 N Salzsäure bei 0°

ein pH von ca. 1,5 eingestellt. Die Reinigung der Titelverbindung erfolgt durch Chromatographie an Opti-Up Kieselgel. $^1$H-NMR (DMSO-$d_6$): δ = 9,30 (d,1H); 8,49 (d,1H); 8,36 (s,1H); 3,57 (m,2H); 1,24 (t,3H).

Beispiel 20: 4-(N-n-Propylamidino)-2-formylpyrimidin-2′-amidinohydrazon-dihydrochlorid

Analog Beispiel 19 erhält man aus 4-Cyano-2-formylpyrimidin-2′-amidinohydrazon-hydrochlorid (Beispiel 17g) nach Behandlung mit Natriummethanolat und n-Propylamin-hydrochlorid die Titelverbindung; $^1$H-NMR (DMSO-$d_6$): δ = 9,29 (d,1H); 8,49 (d,2H); 8,37 (s,1H); 3,53 (m,2H); 1,68 (m,2H); 0,93 (t,3H).

Beispiel 21: 4-(N-Hydroxyamidino)-2-formylpyrimidin-2′-amidinohydrazon-hydrochlorid

Analog Beispiel 19 erhält man aus 4-Cyano-2-formylpyrimidin-2′-amidinohydrazon-hydrochlorid (Beispiel 17g) nach Behandlung mit Natriummethanolat und Hydroxylamin-hydrochlorid die Titelverbindung; $^1$H-NMR (DMSO-$d_6$): δ = 12,67 (s,OH); 8,85 (d,1H); 8,26 (s,1H); 7,82 (d,1H).

Beispiel 22: 1-Hydroxy-3-(2′-amidino-6′-pyridylmethyliden-amino)-guanidin-di-p-toluolsulfonat

Eine Lösung von 2,2 g (0,0076 Mol) 2-Amidino-6-formylpyridin-diethylacetal-hydrochlorid (Beispiel 9b) in 15 ml Methanol wird mit einer Lösung von 1,99 g (0,0076 Mol) 1-Amino-3-hydroxyguanidin-toluolsulfonat in 4 ml Wasser versetzt und nach Zugabe von 3,7 ml 2N Salzsäure 1 h auf 70° erwärmt. Nach dem Abkühlen dampft man ein, nimmt den Rückstand in wenig Ethanol auf, filtriert das ungelöste Material ab und dampft erneut ein. Das Rohprodukt wird mittels Chromatographie an Amberlite XAD 1180 (Wasser als Eluiermittel) gereinigt und anschliessend aus Ethanol/Ether umkristallisiert; Smp. 230-235° (Zersetzung).

Beispiel 23: 2-(N-Methoxyamidino)-6-formylpyridin-2′-amidinohydrazon-dihydrochlorid

Eine Lösung von 0,17 g (0,0076 g-Atom) Natrium in 6 ml abs. Methanol versetzt man bei Raumtemperatur mit 0,85 g (0,0038 Mol) 2-Cyano-6-formylpyridin-2′-amidinohydrazon-hydrochlorid und rührt die rötliche Suspension 12 h bei Raumtemperatur. Der daraus in situ gebildete Iminoether wird mit 0,63 g (0,0076 Mol) O-Methylhydroxylamin-hydrochlorid versetzt und anschliessend weitere 12 h bei Raumtemperatur gerührt. Man filtriert, dampft das Filtrat ein, nimmt den Rückstand in wenig Ethanol auf und filtriert nochmals. Nach Zugabe von ethanolischer Salzsäure kristallisiert die Titelverbindung aus, Smp. 230-234°.

Die Ausgangsverbindung wird wie folgt hergestellt:

(a) 2-Cyano-6-formylpyridin-2′-amidinohydrazon-hydrochlorid

Eine Lösung von 3,09 g (0,022 Mol) Aminoguanidin-hydrogencarbonat in 15 ml Wasser wird tropfenweise mit 22 ml 2 N Salzsäure versetzt ($CO_2$-Entwicklung). Zu dieser Lösung gibt man 3,0 g (0,022 Mol) 2-Cyano-6-formylpyridin gelöst in 15 ml Methanol zu und erhitzt 1 h auf 70°. Nach dem Abkühlen wird die Titelverbindung abfiltriert, mit wenig Methanol/Wasser (1:1) gewaschen und getrocknet; Smp. 295-298°.

Beispiel 24: 2-(N-Aminoamidino)-6-formylpyridin-2′-amidinohydrazon-dihydrochlorid

Analog Beispiel 23 wird der intermediär gebildete Iminoether mit Hydrazin-dihydrochlorid zur Titelverbindung umgesetzt; Smp. 245-248°.

Beispiel 25: 2-(4′,5′-Dihydroimidazol-2′-yl)-6-formylpyridin-2″-amidinohydrazon-dihydrochlorid

Analog Beispiel 23 wird der intermediär gebildete Iminoether mit 1,2-Diaminoethan-dihydrochlorid zur Titelverbindung umgesetzt, Smp. 310-313°.

Beispiel 26: 2-(N-Hydroxyamidino)-6-formylpyridin-2′-amidinohydrazon-tetrahydrochlorid

Analog Beispiel 23 wird der intermediär gebildete Iminoether mit Hydroxylamin-hydrochlorid zur Titelverbindung umgesetzt; Smp. 260-263° (das Produkt enthält 3 Mol Wasser).

Beispiel 27: 1-Amino-3-(2′-amidino-6′-pyridylmethyliden-amino)-guanidin-dihydrochlorid

Analog Beispiel 22 wird 2-Amidino-6-formylpyridin-diethylacetal-hydrochlorid (Beispiel 9b) mit einem 5-fachen Ueberschuss an 1,3-Diaminoguanidin-hydrochlorid zur Titelverbindung umgesetzt; Smp. 250-253°.

Beispiel 28: 1,3-Bis(2′-Amidino-6′-pyridylmethyliden-amino)-guanidin-trihydrochlorid

Analog Beispiel 22 wird 2-Amidino-6-formylpyridin-diethylacetal-hydrochlorid (Beispiel 9b) mit 0,5 Aequivalenten 1,3-Diaminoguanidin-hydrochlorid zur Titelverbindung umgesetzt; Smp. 260-265°.

Beispiel 29: N-(3-Propionylphenyl-2′-amidinohydrazon)-guanidin-dihydrochlorid

Ausgehend von 3-Aminopropiophenon und Aminoguanidin-hydrogencarbonat erhält man via 3-Aminopropiophenon-2′-amidinohydrazon-hydrochlorid analog Beispiel 13 die Titelverbindung.

Beispiel 29a: N-(3-Propionylphenyl-2′-amidinohydrazon)-guanidin-dihydrochlorid

Analog Beispiel 15 wird ausgehend von 3-Guanidinopropiophenon und Aminoguanidin-hydrogencarbonat die Titelverbindung hergestellt.

Beispiel 30: N-(3-Isobutyroylphenyl-2′-amidinohydrazon)-guanidin-dihydrochlorid

Ausgehend von 3-Aminoisobutyrophenon und Aminoguanidin-hydrogencarbonat erhält man via 3-Aminoisobutyrophenon-2′-amidinohydrazon-hydrochlorid analog Beispiel 13 die Titelverbindung.

Beispiel 30a: N-(3-Isobutyroylphenyl-2′-amidinohydrazon)-guanidin-dihydrochlorid

Analog Beispiel 15 wird ausgehend von 3-Guanidinoisobutyrophenon und Aminoguanidin-hydrogencarbonat die Titelverbindung hergestellt.

Beispiel 31: 2-Amidino-6-acetylpyridin-2′-amidinohydrazon-dihydrochlorid

Analog Beispiel 8a und 8 wird ausgehend von 2-Cyano-6-acetylpyridin die Titelverbindung hergestellt.

Beispiel 32: 2-Amidino-6-propionylpyridin-2′-amidinohydrazon-dihydrochlorid

Analog Beispiel 8a und 8 wird ausgehend von 2-Cyano-6-propionylpyridin die Titelverbindung hergestellt.

Beispiel 33: 2-Amidino-6-isobutyroylpyridin-2′-amidinohydrazon-dihydrochlorid

Analog Beispiel 8a und 8 wird ausgehend von 2-Cyano-6-isobutyroylpyridin die Titelverbindung hergestellt.

Beispiel 34: 4-Amidino-2-acetylpyridin-2′-amidinohydrazon-dihydrochlorid

Analog Beispiel 8a und 8 wird ausgehend von 4-Cyano-2-acetylpyridin die Titelverbindung hergestellt.

Beispiel 35: 4-Amidino-2-propionylpyridin-2′-amidinohydrazon-dihydrochlorid

Analog Beispiel 8a und 8 wird ausgehend von 4-Cyano-2-propionylpyridin die Titelverbindung hergestellt.

Beispiel 36: 3-Propionylbenzamidin-2′-amidinohydrazon-dihydrochlorid

Analog Beispiel 3a und 3 wird ausgehend von 3-Propionylbenzonitril die Titelverbindung hergestellt.

Beispiel 37: 3-Isobutyroylbenzamidin-2′-amidinohydrazon-dihydrochlorid

Analog Beispiel 3a und 3 wird ausgehend von 3-Isobutyroylbenzonitril die Titelverbindung hergestellt.

Beispiel 38: 2-Amidino-4-formylpyrimidin-2′-amidinohydrazon-dihydrochlorid

Analog Beispiel 17 wird ausgehend von 2-Cyano-4-formylpyrimidin-2′-amidinohydrazon-hydrochlorid die Titelverbindung erhalten. Das verwendete Ausgangsmaterial wird ausgehend von 2-Cyano-4-methylpyrimidin durch Oxidation mit Selendioxid und Umsetzung des entstehenden 2-Cyano-4-formylpyrimidins mit Aminoguanidin-hydrogencarbonat hergestellt.

Beispiel 39:

Kapseln enthaltend 0,25 g Wirkstoff, z.B. von einer der Verbindungen der Beispiele 1-38, können wie folgt hergestellt werden:

Zusammensetzung (für 5000 Kapseln)

| | |
|---|---|
| Wirkstoff | 1250 g |
| Talk | 180g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

### Patentansprüche für folgende Vertragsstaaten: AT, BE, CH, DE, FR, GB, IT, LI, LU, NL, SE

1. Verbindungen der Formel I

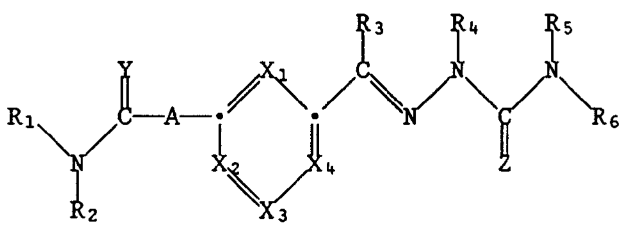

(I),

worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Wasserstoff, Niederalkyl, Hydroxy, verethertes oder verestertes Hydroxy oder unsubstituiertes oder mono- oder disubstituiertes Amino bedeutet; die Reste $R_2$, $R_3$, $R_4$, $R_5$, $R_7$, $R_8$ und $R_9$ unabhängig voneinander Wasserstoff oder Niederalkyl bedeuten, oder $R_1$ und $R_8$ zusammen auch Alkylen bedeuten können; und $R_6$ für Wasserstoff, Niederalkyl, Cycloalkyl, Arylniederalkyl, Aryl, freies oder funktionell abgewandeltes Carboxy, Hydroxy, verethertes oder verestertes Hydroxy, unsubstituiertes oder mono- oder disubstituiertes Amino steht, Tautomere davon, und ihre Salze.

2. Verbindungen der Formel I gemäss Anspruch 1, worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Wasserstoff, Niederalkyl, Hydroxy, Niederalkoxy oder Amino bedeutet; die Reste $R_2$, $R_3$, $R_4$, $R_8$, $R_7$, $R_8$ und $R_9$ unabhängig voneinander Wasserstoff oder Niederalkyl bedeuten, oder $R_1$ und $R_8$ zusammen auch $-(CH_2)_2-$ oder $-(CH_2)_3-$ bedeuten können; und $R_6$ für Wasserstoff, Niederalkyl, Hydroxy, Amino oder eine Gruppe

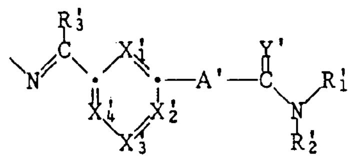

worin A′, $X_1'$, $X_2'$, $X_3'$, $X_4'$, Y′, $R_1'$, $R_2'$ und $R_3'$ wie die entsprechenden Reste A, $X_1$, $X_2$, $X_3$, $X_4$, Y, $R_1$, $R_2$ und $R_3$ definiert sind, steht, Tautomere davon und ihre Salze.

3. Verbindungen der Formel I gemäss Anspruch 1, worin A eine einfache Bindung oder eine Gruppe NH bedeutet; die Reste $X_1$, $X_2$, $X_3$ und $X_4$ folgende Bedeutungen haben:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_4 = N, X_1 = X_2 = X_3 = CH$
$X_1 = X_2 = N, X_3 = X_4 = CH$
$X_1 = X_3 = N, X_2 = X_4 = CH$
$X_1 = X_4 = N, X_2 = X_3 = CH$;
Y für $NR_8$ oder auch - falls A eine einfache Bindung bedeutet - für O steht; Z für $NR_9$, O oder S steht; $R_1$ Wasserstoff, Niederalkyl, Hydroxy, Niederalkoxy oder Amino bedeutet; die Reste $R_2$, $R_3$, $R_8$ und $R_9$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_8$ für Wasserstoff, Niederalkyl, Hydroxy, Amino, 3-Amidinophenylmethylidenamino oder 2-Amidino-6-pyridylmethylidenamino steht, Tautomere davon, und ihre Salze.

4. Verbindungen der Formel I gemäss Anspruch 1, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für $NR_8$ oder auch - falls A eine einfache Bindung bedeutet - für O steht; Z für NH oder S steht; $R_1$ Wasserstoff, Niederalkyl, Hydroxy, Niederalkoxy oder Amino bedeutet; die Reste $R_2$, $R_3$ und $R_8$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_8$ für Wasserstoff, Niederalkyl, Hydroxy, Amino, 3-Amidinophenylmethylidenamino oder 2-Amidino-6-pyridylmethylidenamino steht, Tautomere davon, und ihre Salze.

5. Verbindungen der Formel I gemäss Anspruch 1, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für NH oder auch - falls A eine einfache Bindung bedeutet - für O steht; Z für NH oder S steht; $R_1$ Wasserstoff, Niederalkyl, Hydroxy, Niederalkoxy oder Amino bedeutet; $R_3$ Wasserstoff oder Niederalkyl bedeutet; $R_2$, $R_4$ und $R_5$ Wasserstoff bedeuten und $R_6$ für Wasserstoff, Niederalkyl, Hydroxy oder Amino steht, Tautomere davon, und ihre Salze.

6. Verbindungen der Formel I gemäss Anspruch 1, worin A eine einfache Bindung oder eine Gruppe $NR_7$ bedeutet; $X_1$, $X_2$, $X_3$ und $X_4$ unabhängig voneinander CH oder HN bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; die Reste $R_1$, $R_2$, $R_3$, $R_4$, $R_5$, $R_7$, $R_8$ und $R_9$ unabhängig voneinander Wasserstoff oder Niederalkyl bedeuten und $R_6$ für Wasserstoff, Niederalkyl, Hydroxy, Amino oder 3-Amidinophenylmethylidenamino steht, Tautomere davon, und ihre Salze.

7. Verbindungen der Formel I gemäss Anspruch 1, worin A eine einfache Bindung oder eine Gruppe NH bedeutet; die Reste $X_1$, $X_2$, $X_3$ und $X_4$ folgende Bedeutungen haben:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$

$X_1 = X_2 = N, X_3 = X_4 = CH$
$X_1 = X_3 = N, X_2 = X_4 = CH$
$X_1 = X_4 = N, X_2 = X_3 = CH$;
Y für $NR_8$ oder auch - falls A eine einfache Bindung bedeutet - für O steht; Z für $NR_9$, O oder S steht; die Reste $R_1$, $R_2$, $R_3$, $R_8$ und $R_9$ unabhängig voneinander Wasserstoff oder Niederalkyl bedeuten, $R_4$ und $R_8$ Wasserstoff bedeuten und $R_8$ für Wasserstoff, Niederalkyl, Hydroxy, Amino, oder 3-Amidinophenylmethylidenamino, Tautomere davon, und ihre Salze.

**8.** Verbindungen der Formel I gemäss Anspruch 1, worin A eine einfache Bindung oder eine Gruppe NH bedeutet; $X_1$ und $X_2$ unabhängig voneinander CH oder N bedeuten mit der Massgabe, dass mindestens eine der Gruppen $X_1$ und $X_2$ CH bedeutet; $X_3$ und $X_4$ für CH stehen; Y für NH oder auch - falls A eine einfache Bindung bedeutet - für O steht; Z für NH oder S steht; der Rest $R_3$ Wasserstoff oder Niederalkyl bedeutet; $R_1$, $R_2$, $R_4$ und $R_8$ Wasserstoff bedeuten und $R_8$ für Wasserstoff, Niederalkyl, Hydroxy oder Amino steht, Tautomere davon, und ihre Salze.

**9.** 3-Formylbenzamidin-2′-amidinohydrazon gemäss Anspruch 1 oder ein pharmazeutisch verwendbares Salz davon.

**10.** 3-Acetylbenzamidin-2′-amidinohydrazon gemäss Anspruch 1 oder ein pharmazeutisch verwendbares Salz davon.

**11.** 1,3-Bis(3′-Amidinobenzyliden-amino)-guanidin gemäss Anspruch 1 oder ein pharmazeutisch verwendbares Salz davon.

**12.** 2-Amidino-6-formylpyridin-2′-amidinohydrazon gemäss Anspruch 1 oder ein pharmazeutisch verwendbares Salz davon.

**13.** 1-Hydroxy-3(2′-amidino-6-pyridylmethyliden-amino)-guanidin gemäss Anspruch 1 oder ein pharmazeutisch verwendbares Salz davon.

**14.** 3-Formylbenzamidin-2′-(N-hydroxyamidino)-hydrazon gemäss Anspruch 1 oder ein pharmazeutisch verwendbares Salz hiervon

**15.** Pharmazeutische Präparate enthaltend eine Verbindung gemäss einem der Ansprüche 1 bis 14 und mindestens ein pharmazeutisch verwendbares Trägermaterial.

**16.** Eine Verbindung gemäss einem der Ansprüche 1 bis 14 zur Anwendung in einem Verfahren zur therapeutischen Behandlung des tierischen oder menschlichen Körpers.

**17.** Verwendung von einer Verbindung gemäss einem der Ansprüche 1 bis 14 zur Herstellung eines pharmazeutischen Präparats zur Anwendung in einem Verfahren zur therapeutischen Behandlung von Tumoren.

**18.** Verfahren zur Herstellung einer Verbindung der Formel I gemäss Anspruch 1, dadurch gekennzeichnet, dass man
a) eine Verbindung der Formel II

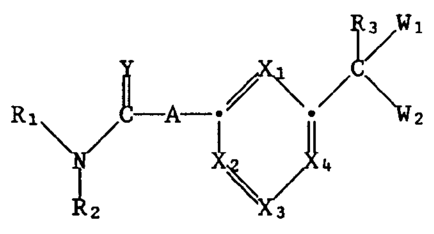

(II)

worin die Gruppe $CW_1W_2$ für Carbonyl, funktionell abgewandeltes Carbonyl oder geschütztes Carbonyl steht, mit einem Amin der Formel III

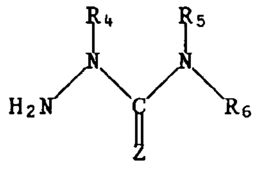

(III)

kondensiert, oder

b) zur Herstellung einer Verbindung der Formel I, worin A eine Gruppe $-NR_7-$ bedeutet, eine Verbindung der Formel IV

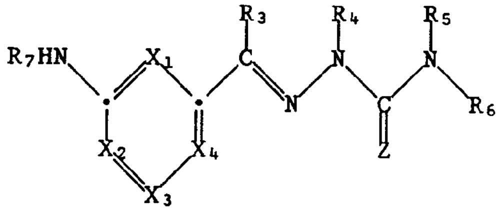

(IV)

mit einem Reagens umsetzt, das geeignet ist, die Gruppe $-NHR_7$ in ein Guanidin der Formel I umzuwandeln, oder

c) zur Herstellung einer Verbindung der Formel I, worin A eine einfache Bindung bedeutet, in einer Verbindung der Formel VI

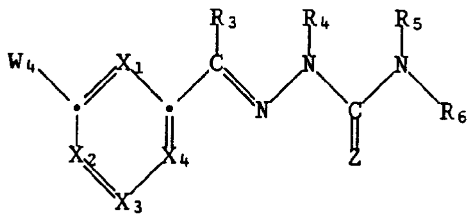

(VI)

worin $W_4$ einen Rest, der in ein Amidin bzw. eine Carbamoylverbindung der Formel I überführt werden kann, bedeutet, den Rest $W_4$ in die Gruppe $-C(=Y)NR_1R_2$ überführt; wobei in den obigen Ausgangsstoffen der Formeln II bis VI die Symbole A, $X_1$, $X_2$, $X_3$, $X_4$, Y, Z und $R_1$-$R_7$ die unter Formel I angegebene Bedeutung haben; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

**Patentansprüche für folgende Vertragsstaaten : ES, GR**

1. Verfahren zur Herstellung von Verbindungen der Formel I

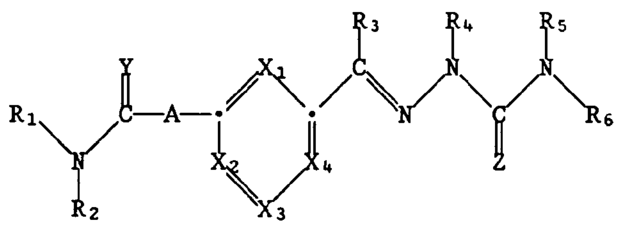

(I),

worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Was-

serstoff, Niederalkyl, Hydroxy, verethertes oder verestertes Hydroxy oder unsubstituiertes oder mono- oder disubstituiertes Amino bedeutet; die Reste $R_2$, $R_3$, $R_4$, $R_5$, $R_7$, $R_8$ und $R_9$ unabhängig voneinander Wasserstoff oder Niederalkyl bedeuten, oder $R_1$ und $R_8$ zusammen auch Alkylen bedeuten können; und $R_6$ für Wasserstoff, Niederalkyl, Cycloalkyl, Arylniederalkyl, Aryl, freies oder funktionell abgewandeltes Carboxy, Hydroxy, verethertes oder verestertes Hydroxy, unsubstituiertes oder mono- oder disubstituiertes Amino steht, von Tautomeren davon, und von ihren Salzen, dadurch gekennzeichnet, dass man

a) eine Verbindung der Formel II

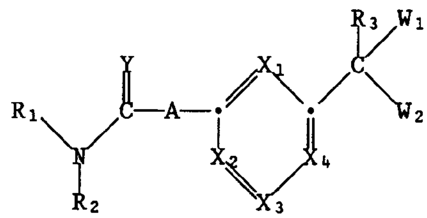

(II)

worin die Gruppe $CW_1W_2$ für Carbonyl, funktionell abgewandeltes Carbonyl oder geschütztes Carbonyl steht, mit einem Amin der Formel III

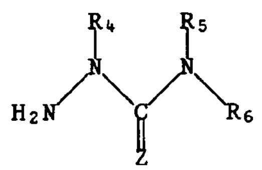

(III)

kondensiert, oder

b) zur Herstellung einer Verbindung der Formel I, worin A eine Gruppe $-NR_7-$ bedeutet, eine Verbindung der Formel IV

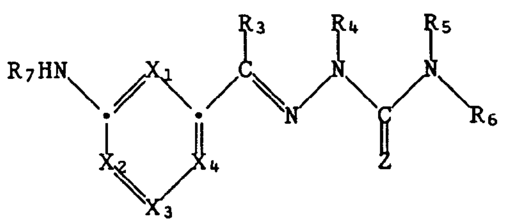

(IV)

mit einem Reagens umsetzt, das geeignet ist, die Gruppe $-NHR_7$ in ein Guanidin der Formel I umzuwandeln, oder

c) zur Herstellung einer Verbindung der Formel I, worin A eine einfache Bindung bedeutet, in einer Verbindung der Formel VI

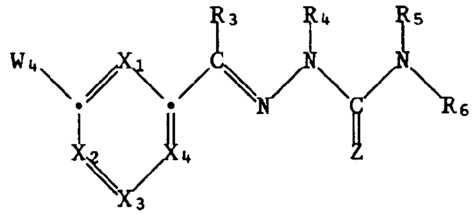

(VI)

worin $W_4$ einen Rest, der in ein Amidin bzw. eine Carbamoylverbindung der Formel I überführt werden kann, bedeutet, den Rest $W_4$ in die Gruppe $-C(=Y)NR_1R_2$ überführt; wobei in den obigen Ausgangsstoffen der Formeln II bis VI die Symbole A, $X_1$, $X_2$, $X_3$, $X_4$, Y, Z und $R_1$-$R_7$ die unter Formel I angegebene Bedeutung haben; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

2. Verfahren gemäss Anspruch 1 zur Herstellung von Verbindungen der Formel I, worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; $R_1$ Wasserstoff, Niederalkyl, Hydroxy, Niederalkoxy oder Amino bedeutet; die Reste $R_2$, $R_3$, $R_4$, $R_5$, $R_7$, $R_8$ und $R_9$ unabhängig voneinander Wasserstoff oder Niederalkyl bedeuten, oder $R_1$ und $R_8$ zusammen auch $-(CH_2)_2-$ oder $-(CH_2)_3-$ bedeuten können; und $R_8$ für Wasserstoff, Niederalkyl, Hydroxy, Amino oder eine Gruppe

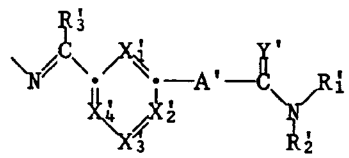

worin A′, $X_1'$, $X_2'$, $X_3'$, $X_4'$, Y′, $R_1'$, $R_2'$ und $R_3'$ wie die entsprechenden Reste A, $X_1$, $X_2$, $X_3$, $X_4$, Y, $R_1$, $R_2$ und $R_3$ definiert sind, steht, von Tautomeren davon und ihren Salzen, dadurch gekennzeichnet, dass man entsprechend substituierte Ausgangsverbindungen verwendet.

3. Verfahren gemäss Anspruch 1 zur Herstellung von Verbindungen der Formel I, worin A eine einfache Bindung oder eine Gruppe NH bedeutet; die Reste $X_1$, $X_2$, $X_3$ und $X_4$ folgende Bedeutungen haben:
$X_1 = X_2 = X_3 = X_4 = CH$
$X_1 = N$, $X_2 = X_3 = X_4 = CH$
$X_2 = N$, $X_1 = X_3 = X_4 = CH$
$X_3 = N$, $X_1 = X_2 = X_4 = CH$
$X_4 = N$, $X_1 = X_2 = X_3 = CH$
$X_1 = X_2 = N$, $X_3 = X_4 = CH$
$X_1 = X_3 = N$, $X_2 = X_4 = CH$
$X_1 = X_4 = N$, $X_2 = X_3 = CH$;
Y für $NR_8$ oder auch - falls A eine einfache Bindung bedeutet - für O steht; Z für $NR_9$, O oder S steht; $R_1$ Wasserstoff, Niederalkyl, Hydroxy, Niederalkoxy oder Amino bedeutet; die Reste $R_2$, $R_3$, $R_8$ und $R_9$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_8$ für Wasserstoff, Niederalkyl, Hydroxy, Amino, 3-Amidinophenylmethylidenamino oder 2-Amidino-6-pyridylmethylidenamino steht, von Tautomeren davon, und von ihren Salzen, dadurch gekennzeichnet, dass man entsprechend substituierte Ausgangsverbindungen verwendet.

4. Verfahren gemäss Anspruch 1 zur Herstellung von Verbindungen der Formel I, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für $NR_8$ oder auch - falls A eine einfache Bindung bedeutet - für O steht; Z für NH oder S steht; $R_1$ Wasserstoff, Niederalkyl, Hydroxy, Niederalkoxy oder Amino bedeutet; die Reste $R_2$, $R_3$ und $R_8$ unabhängig voneinander Wasserstoff oder Niederalkyl bedeuten, oder $R_1$ und $R_8$ zusammen auch $-(CH_2)_2-$ bedeuten können; $R_4$ und $R_5$ Wasserstoff bedeuten und $R_6$ für Wasserstoff, Niederalkyl, Hydroxy, Amino, 3-Amidinophenylmethylidenamino oder 2-Amidino-6-pyridylmethylidenamino steht, von Tautomeren davon, und von ihren Salzen, dadurch gekennzeichnet, dass man entsprechend substituierte Ausgangsverbindungen verwendet.

5. Verfahren gemäss Anspruch 1 zur Herstellung von Verbindungen der Formel I, worin A eine einfache Bindung oder eine Gruppe NH bedeutet; worin (a) $X_1$, $X_2$, $X_3$ und $X_4$ CH bedeuten, oder (b) $X_1$ für N steht und $X_2$, $X_3$ und $X_4$ CH bedeuten, oder (c) $X_2$ für N steht und $X_1$, $X_3$ und $X_4$ CH bedeuten, oder (d) $X_1$ und $X_4$ für N stehen und $X_2$ und $X_3$ CH bedeuten; Y für NH oder auch - falls A eine einfache Bindung bedeutet - für O steht; Z für NH oder S steht; $R_1$ Wasserstoff, Niederalkyl, Hydroxy, Niederalkoxy oder Amino bedeutet; $R_3$ Wasserstoff oder Niederalkyl bedeutet; $R_2$, $R_4$ und $R_8$ Wasserstoff bedeuten und $R_6$ für Wasserstoff,

Niederalkyl, Hydroxy oder Amino steht, von Tautomeren davon, und von ihren Salzen, dadurch gekennzeichnet, dass man entsprechend substituierte Ausgangsverbindungen verwendet.

6. Verfahren gemäss Anspruch 1 zur Herstellung von Verbindungen der Formel I, worin A eine einfache Bindung oder eine Gruppe $NR_7$ bedeutet; $X_1$, $X_2$, $X_3$ und $X_4$ unabhängig voneinander CH oder N bedeuten mit der Massgabe, dass mindestens zwei der Gruppen $X_1$-$X_4$ CH bedeuten; Y für $NR_8$ oder auch - falls A eine einfache Bindung bedeutet - für O steht; Z für $NR_9$, O oder S steht; die Reste $R_1$, $R_2$, $R_3$, $R_4$, $R_5$, $R_7$, $R_8$ und $R_9$ unabhängig voneinander Wasserstoff oder Niederalkyl bedeuten und $R_8$ für Wasserstoff, Niederalkyl, Hydroxy, Amino oder 3-Amidinophenylmethylidenamino steht, von Tautomeren davon, und von ihren Salzen, dadurch gekennzeichnet, dass man entsprechend substituierte Ausgangsverbindungen verwendet.

7. Verfahren gemäss Anspruch 1 zur Herstellung von Verbindungen der Formel I, worin A eine einfache Bindung oder eine Gruppe NH bedeutet; die Reste $X_1$, $X_2$, $X_3$ und $X_4$ folgende Bedeutungen haben:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$;
Y für $NR_8$ oder auch - falls A eine einfache Bindung bedeutet - für O steht; Z für $NR_9$, O oder S steht; die Reste $R_1$, $R_2$, $R_3$, $R_8$ und $R_9$ unabhängig voneinander Wasserstoff oder Niederalkyl bedeuten, $R_4$ und $R_8$ Wasserstoff bedeuten und $R_8$ für Wasserstoff, Niederalkyl, Hydroxy, Amino, oder 3-Amidinophenylmethylidenamino, von Tautomeren davon, und von ihren Salzen, dadurch gekennzeichnet, dass man entsprechend substituierte Ausgangsverbindungen verwendet.

8. Verfahren gemäss Anspruch 1 zur Herstellung von Verbindungen der Formel I, worin A eine einfache Bindung oder eine Gruppe NH bedeutet; $X_1$ und $X_2$ unabhängig voneinander CH oder N bedeuten mit der Massgabe, dass mindestens eine der Gruppen $X_1$ und $X_2$ CH bedeutet; $X_3$ und $X_4$ für CH stehen; Y für NH oder auch - falls A eine einfache Bindung bedeutet - für O steht; Z für NH oder S steht; der Rest $R_3$ Wasserstoff oder Niederalkyl bedeutet; $R_1$, $R_2$, $R_4$ und $R_5$ Wasserstoff bedeuten und $R_8$ für Wasserstoff, Niederalkyl, Hydroxy oder Amino steht, von Tautomeren davon, und von ihren Salzen, dadurch gekennzeichnet, dass man entsprechend substituierte Ausgangsverbindungen verwendet.

9. Verfahren gemäss Anspruch 1 zur Herstellung von 3-Formylbenzamidin-2′-amidinohydrazon oder einem pharmazeutisch verwendbaren Salz davon, dadurch gekennzeichnet, dass man entsprechend substituierte Ausgangsverbindungen verwendet.

10. Verfahren gemäss Anspruch 1 zur Herstellung von 3-Acetylbenzamidin-2′-amidinohydrazon oder einem pharmazeutisch verwendbaren Salz davon, dadurch gekennzeichnet, dass man entsprechend substituierte Ausgangsverbindungen verwendet.

11. Verfahren gemäss Anspruch 1 zur Herstellung von 1,3-Bis(3′-Amidinobenzyliden-amino)-guanidin oder einem pharmazeutisch verwendbaren Salz davon, dadurch gekennzeichnet, dass man entsprechend substituierte Ausgangsverbindungen verwendet.

12. Verfahren gemäss Anspruch 1 zur Herstellung von 2-Amidino-6-formylpyridin-2′-amidinohydrazon oder einem pharmazeutisch verwendbaren Salz davon, dadurch gekennzeichnet, dass man entsprechend substituierte Ausgangsverbindungen verwendet.

13. Verfahren gemäss Anspruch 1 zur Herstellung von 1-Hydroxy-3-(2′-amidino-6′-pyridylmethylidenamino)-guanidin oder einem pharmazeutisch verwendbaren Salz davon, dadurch gekennzeichnet, dass man entsprechend substituierte Ausgangsverbindungen verwendet.

14. Verfahren gemäss Anspruch 1 zur Herstellung von 3-Formylbenzamidin-2′-(N-hydroxyamidino)-hydrazon oder einem pharmazeutisch verwendbaren Salz hiervon, dadurch gekennzeichnet, dass man entsprechend substituierte Ausgangsverbindungen verwendet.

15. Verfahren zur Herstellung von pharmazeutischen Präparaten enthaltend eine gemäss einem Verfahren aus einem der Ansprüche 1 bis 14 hergestellte Verbindung der Formel I und mindestens ein pharmazeutisch verwendbares Trägermaterial, dadurch gekennzeichnet, dass man eine solche Verbindung der Formel I mit mindestens einem pharmazeutisch verwendbaren Trägermaterial mischt.

## Claims

### Claims for the following Contracting States : AT, BE, CH, DE, FR, GB, IT, LI, LU, NL, SE

1. A compound of formula I

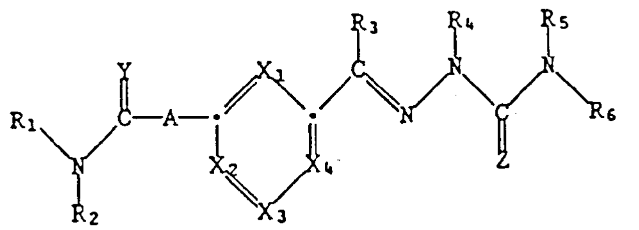

(I)

wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; $R_1$ is hydrogen, lower alkyl, hydroxy, etherified or esterified hydroxy, or unsubstituted or mono- or di-substituted amino; each of the radicals $R_2$, $R_3$, $R_4$, $R_5$, $R_7$, $R_8$ and $R_9$, independently of the others, is hydrogen or lower alkyl, or $R_1$ and $R_8$ together may also be alkylene; and $R_8$ is hydrogen, lower alkyl, cycloalkyl, aryl-lower alkyl, aryl, free or funtionally modified carboxy, hydroxy, etherified or esterified hydroxy, unsubstituted or mono- or di-substituted amino, tautomers thereof, or a salt thereof.

2. A compound of formula I according to claim 1, wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; $R_1$ is hydrogen, lower alkyl, hydroxy, lower alkoxy or amino; each of the radicals $R_2$, $R_3$, $R_4$, $R_8$, $R_7$, $R_8$ and $R_9$, independently of the others, is hydrogen or lower alkyl, or $R_1$ and $R_8$ together may also be $-(CH_2)_2-$ or $-(CH_2)_3-$; and $R_8$ is hydrogen, lower alkyl, hydroxy, amino or a group

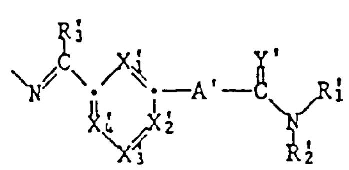

wherein A′, $X_1'$, $X_2'$, $X_3'$, $X_4'$, Y′, $R_1'$, $R_2'$ and $R_3'$ have the same definitions as the corresponding radicals A, $X_1$, $X_2$, $X_3$, $X_4$, Y, $R_1$, $R_2$ and $R_3$, tautomers thereof, or a salt thereof,

3. A compound of formula I according to claim 1, wherein A is a single bond or an NH group; the radicals $X_1$, $X_2$, $X_3$ and $X_4$ have the following meanings:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_4 = N, X_1 = X_2 = X_3 = CH$
$X_1 = X_2 = N, X_3 = X_4 = CH$
$X_1 = X_3 = N, X_2 = X_4 = CH$
$X_1 = X_4 = N, X_2 = X_3 = CH$;
Y is $NR_8$ or also, if A is a single bond, is O; Z is $NR_9$, O or S; $R_1$ is hydrogen, lower alkyl, hydroxy, lower

alkoxy or amino; each of the radicals $R_2$, $R_3$, $R_8$ and $R_9$, independently of the others, is hydrogen or lower alkyl, or $R_1$ and $R_8$ together may also be $-(CH_2)_2-$; $R_4$ and $R_5$ are hydrogen, and $R_8$ is hydrogen, lower alkyl, hydroxy, amino, 3-amidinophenylmethylideneamino or 2-amidino-6-pyridylmethylideneamino, tautomers thereof, or a salt thereof.

**4.** A compound of formula I according to claim 1, wherein A is a single bond or an NH group; wherein (a) $X_1$, $X_2$, $X_3$ and $X_4$ are CH, or (b) $X_1$ is N and $X_2$, $X_3$ and $X_4$ are CH, or (c) $X_2$ is N and $X_1$, $X_3$ and $X_4$ are CH, or (d) $X_1$ and $X_4$ are N and $X_2$ and $X_3$ are CH; Y is $NR_8$ or also, if A is a single bond, is O; Z is NH or S; $R_1$ is hydrogen, lower alkyl, hydroxy, lower alkoxy or amino; each of the radicals $R_2$, $R_3$ and $R_8$, independently of the others, is hydrogen or lower alkyl, or $R_1$ and $R_8$ together may also be $-(CH_2)_2-$; $R_4$ and $R_5$ are hydrogen, and $R_6$ is hydrogen, lower alkyl, hydroxy, amino, 3-amidinophenylmethylideneamino or 2-amidino-6-pyridylmethylideneamino, tautomers thereof, or a salt thereof.

**5.** A compound of formula I according to claim 1, wherein A is a single bond or an NH group; wherein (a) $X_1$, $X_2$, $X_3$ and $X_4$ are CH, or (b) $X_1$ is N and $X_2$, $X_3$ and $X_4$ are CH, or (c) $X_2$ is N and $X_1$, $X_3$ and $X_4$ are CH, or (d) $X_1$ and $X_4$ are N and $X_2$ and $X_3$ are CH; Y is NH or also, if A is a single bond, is O; Z is NH or S; $R_1$ is hydrogen, lower alkyl, hydroxy, lower alkoxy or amino; $R_3$ is hydrogen or lower alkyl; $R_2$, $R_4$ and $R_8$ are hydrogen, and $R_8$ is hydrogen, lower alkyl, hydroxy or amino, tautomers thereof, or a salt thereof.

**6.** A compound of formula I according to claim 1, wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; each of the radicals $R_1$, $R_2$, $R_3$, $R_4$, $R_5$, $R_7$, $R_8$ and $R_9$, independently of the others, is hydrogen or lower alkyl, and $R_8$ is hydrogen, lower alkyl, hydroxy, amino or 3-amidinophenylmethylideneamino, tautomers thereof, or a salt thereof.

**7.** A compound of formula I according to claim 1, wherein A is a single bond or an NH group; the radicals $X_1$, $X_2$, $X_3$ and $X_4$ have the following meanings:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$;
Y is $NR_8$ or also, if A is a single bond, is O; Z is $NR_9$, O or S; each of the radicals $R_1$, $R_2$, $R_3$, $R_8$ and $R_9$, independently of the others, is hydrogen or lower alkyl, $R_4$ and $R_8$ are hydrogen, and $R_8$ is hydrogen, lower alkyl, hydroxy, amino or 3-amidinophenylmethylideneamino, tautomers thereof, or a salt thereof.

**8.** A compound of formula I according to claim 1, wherein A is a single bond or an NH group; each of $X_1$ and $X_2$, independently of the other, is CH or N, with the proviso that at least one of the groups $X_1$ and $X_2$ is CH; $X_3$ and $X_4$ are CH; Y is NH or also, if A is a single bond, is O; Z is NH or S; the radical $R_3$ is hydrogen or lower alkyl; $R_1$, $R_2$, $R_4$ and $R_5$ are hydrogen, and $R_6$ is hydrogen, lower alkyl, hydroxy or amino, tautomers thereof, or a salt thereof.

**9.** 3-Formylbenzamidine-2′-amidinohydrazone according to claim 1 or a pharmaceutically acceptable salt thereof.

**10.** 3-Acetylbenzamidine-2′-amidinohydrazone according to claim 1 or a pharmaceutically acceptable salt thereof.

**11.** 1,3-Bis(3′-amidinobenzylidene-amino)guanidine according to claim 1 or a pharmaceutically acceptable salt thereof.

**12.** 2-Amidino-6-formylpyridine-2′-amidinohydrazone according to claim 1 or a pharmaceutically acceptable salt thereof.

**13.** 1-Hydroxy-3-(2′-amidino-6′-pyridylmethylidene-amino)guanidine according to claim 1 or a pharmaceutically acceptable salt thereof.

**14.** 3-Formylbenzamidine-2'-(N-hydroxyamidino)hydrazone according to claim 1 or a pharmaceutically acceptable salt thereof.

**15.** A pharmaceutical composition comprising a compound according to any one of claims 1 to 14 and at least one pharmaceutically acceptable carrier.

**16.** A compound according to any one of claims 1 to 14 for use in a method for the therapeutic treatment of the animal or human body.

**17.** The use of a compound according to any one of claims 1 to 14 for the preparation of a pharmaceutical composition for use in a method for the therapeutic treatment of tumours.

**18.** A process for the preparation of a compound of formula I according to claim 1, which comprises
a) condensing a compound of formula II

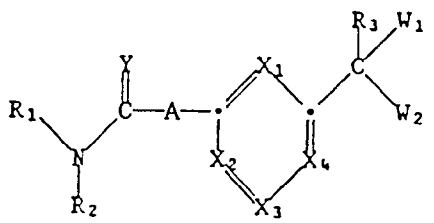

(II),

wherein the group $CW_1W_2$ is carbonyl, functionally modified carbonyl or protected carbonyl, with an amine of formula III

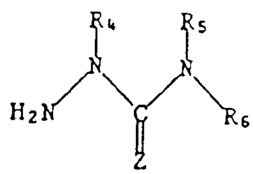

(III),

or
b) for the preparation of a compound of formula I wherein A is a group $-NR_7-$, reacting a compound of formula IV

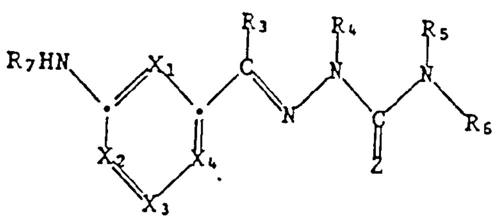

(IV)

with a reagent suitable for converting the group $-NHR_7$ into a guanidine of formula I, or
c) for the preparation of a compound of formula I wherein A is a single bond, in a compound of formula VI

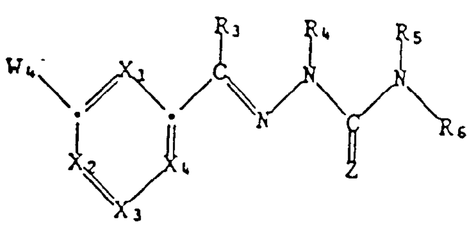

(VI),

wherein $W_4$ is a radical that can be converted into an amidine or a carbamoyl compound of formula I, converting the radical $W_4$ into the group $-C(=Y)NR_1R_2$; the symbols A, $X_1$, $X_2$, $X_3$, $X_4$, Y, Z and $R_1$ to $R_7$ in the above starting materials of formulae II to VI being as defined for formula I; and, if desired, converting a resulting compound of formula I into another compound of formula I, and/or, if desired, converting a resulting salt into the free compound or into another salt, and/or, if desired, converting a resulting free compound of formula I having salt-forming properties into a salt.

**Claims for the following Contracting States : ES, GR**

1. A process for the preparation of a compound of formula I

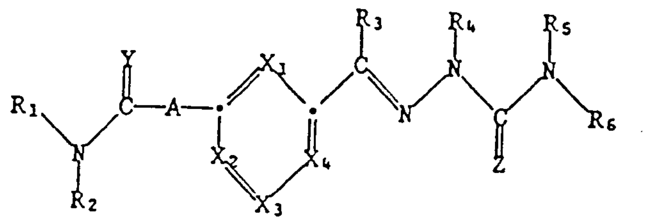

(I)

wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; $R_1$ is hydrogen, lower alkyl, hydroxy, etherified or esterified hydroxy, or unsubstituted or mono- or di-substituted amino; each of the radicals $R_2$, $R_3$, $R_4$, $R_5$, $R_7$, $R_8$ and $R_9$, independently of the others, is hydrogen or lower alkyl, or $R_1$ and $R_8$ together may also be alkylene; and $R_6$ is hydrogen, lower alkyl, cycloalkyl, aryl-lower alkyl, aryl, free or funtionally modified carboxy, hydroxy, etherified or esterified hydroxy, unsubstituted or mono- or di-substituted amino, tautomers thereof, or a salt thereof, which process comprises

a) condensing a compound of formula II

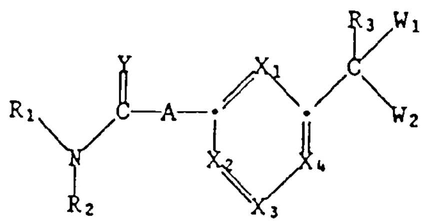

(II),

wherein the group $CW_1W_2$ is carbonyl, functionally modified carbonyl or protected carbonyl, with an amine of formula III

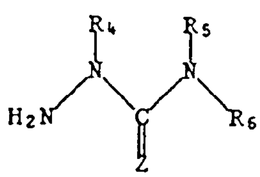

(III),

or

b) for the preparation of a compound of formula I wherein A is a group $-NR_7-$, reacting a compound of formula IV

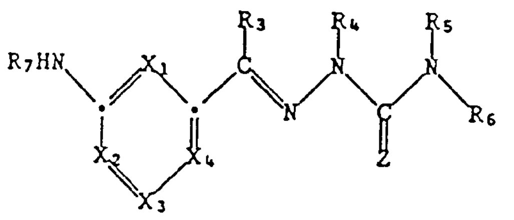

(IV)

with a reagent suitable for converting the group $-NHR_7$ into a guanidine of formula I, or

c) for the preparation of a compound of formula I wherein A is a single bond, in a compound of formula VI

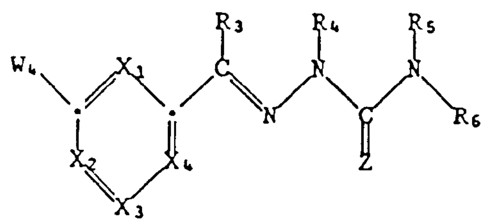

(VI),

wherein $W_4$ is a radical that can be converted into an amidine or a carbamoyl compound of formula I, converting the radical $W_4$ into the group $-C(=Y)NR_1R_2$; the symbols A, $X_1$, $X_2$, $X_3$, $X_4$, Y, Z and $R_1$ to $R_7$ in the above starting materials of formulae II to VI being as defined for formula I; and, if desired, converting a resulting compound of formula I into another compound of formula I, and/or, if desired, converting a resulting salt into the free compound or into another salt, and/or, if desired, converting a resulting free compound of formula I having salt-forming properties into a salt.

**2.** A process according to claim 1 for the preparation of a compound of formula I wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; $R_1$ is hydrogen, lower alkyl, hydroxy, lower alkoxy or amino; each of the radicals $R_2$, $R_3$, $R_4$, $R_5$, $R_7$, $R_8$ and $R_9$, independently of the others, is hydrogen or lower alkyl, or $R_1$ and $R_8$ together may also be $-(CH_2)_2-$ or $-(CH_2)_3-$; and $R_6$ is hydrogen, lower alkyl, hydroxy, amino or a group

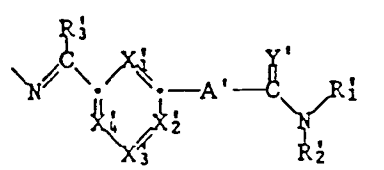

wherein A′, $X_1'$, $X_2'$, $X_3'$, $X_4'$, Y′, $R_1'$, $R_2'$ and $R_3'$ have the same definitions as the corresponding radicals A, $X_1$, $X_2$, $X_3$, $X_4$, Y, $R_1$, $R_2$ and $R_3$, tautomers thereof, or a salt thereof, wherein correspondingly substituted starting compounds are used.

**3.** A process according to claim 1 for the preparation of a compound of formula I wherein A is a single bond or an NH group; the radicals $X_1$, $X_2$, $X_3$ and $X_4$ have the following meanings:

$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_4 = N, X_1 = X_2 = X_3 = CH$
$X_1 = X_2 = N, X_3 = X_4 = CH$
$X_1 = X_3 = N, X_2 = X_4 = CH$
$X_1 = X_4 = N, X_2 = X_3 = CH$;

Y is $NR_8$ or also, if A is a single bond, is O; Z is $NR_9$, O or S; $R_1$ is hydrogen, lower alkyl, hydroxy, lower alkoxy or amino; each of the radicals $R_2$, $R_3$, $R_8$ and $R_9$, independently of the others, is hydrogen or lower

alkyl, or $R_1$ and $R_8$ together may also be $-(CH_2)_2-$; $R_4$ and $R_5$ are hydrogen, and $R_8$ is hydrogen, lower alkyl, hydroxy, amino, 3-amidinophenylmethylideneamino or 2-amidino-6-pyridylmethylideneamino, tautomers thereof, or a salt thereof, wherein correspondingly substituted starting compounds are used.

**4.** A process according to claim 1 for the preparation of a compound of formula I wherein A is a single bond or an NH group; wherein (a) $X_1$, $X_2$, $X_3$ and $X_4$ are CH, or (b) $X_1$ is N and $X_2$, $X_3$ and $X_4$ are CH, or (c) $X_2$ is N and $X_1$, $X_3$ and $X_4$ are CH, or (d) $X_1$ and $X_4$ are N and $X_2$ and $X_3$ are CH; Y is $NR_8$ or also, if A is a single bond, is O; Z is NH or S; $R_1$ is hydrogen, lower alkyl, hydroxy, lower alkoxy or amino; each of the radicals $R_2$, $R_3$ and $R_8$, independently of the others, is hydrogen or lower alkyl, or $R_1$ and $R_8$ together may also be $-(CH_2)_2-$; $R_4$ and $R_8$ are hydrogen, and $R_6$ is hydrogen, lower alkyl, hydroxy, amino, 3-amidinophenylmethylideneamino or 2-amidino-6-pyridylmethylideneamino, tautomers thereof, or a salt thereof, wherein correspondingly substituted starting compounds are used.

**5.** A process according to claim 1 for the preparation of a compound of formula I wherein A is a single bond or an NH group; wherein (a) $X_1$, $X_2$, $X_3$ and $X_4$ are CH, or (b) $X_1$ is N and $X_2$, $X_3$ and $X_4$ are CH, or (c) $X_2$ is N and $X_1$, $X_3$ and $X_4$ are CH, or (d) $X_1$ and $X_4$ are N and $X_2$ and $X_3$ are CH; Y is NH or also, if A is a single bond, is O; Z is NH or S; $R_1$ is hydrogen, lower alkyl, hydroxy, lower alkoxy or amino; $R_3$ is hydrogen or lower alkyl; $R_2$, $R_4$ and $R_5$ are hydrogen, and $R_8$ is hydrogen, lower alkyl, hydroxy or amino, tautomers thereof, or a salt thereof, wherein correspondingly substituted starting compounds are used.

**6.** A process according to claim 1 for the preparation of a compound of formula I wherein A is a single bond or a group $NR_7$; each of $X_1$, $X_2$, $X_3$ and $X_4$, independently of the others, is CH or N, with the proviso that at least two of the groups $X_1$ to $X_4$ are CH; Y is $NR_8$ or also, if A is a single bond, is O; Z is $NR_9$, O or S; each of the radicals $R_1$, $R_2$, $R_3$, $R_4$, $R_5$, $R_7$, $R_8$ and $R_9$, independently of the others, is hydrogen or lower alkyl, and $R_8$ is hydrogen, lower alkyl, hydroxy, amino or 3-amidinophenylmethylideneamino, tautomers thereof, or a salt thereof, wherein correspondingly substituted starting compounds are used.

**7.** A process according to claim 1 for the preparation of a compound of formula I wherein A is a single bond or an NH group; the radicals $X_1$; $X_2$, $X_3$ and $X_4$ have the following meanings:
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$;
Y is $NR_8$ or also, if A is a single bond, is O; Z is $NR_9$, O or S; each of the radicals $R_1$, $R_2$, $R_3$, $R_8$ and $R_9$, independently of the others, is hydrogen or lower alkyl, $R_4$ and $R_8$ are hydrogen, and $R_8$ is hydrogen, lower alkyl, hydroxy, amino or 3-amidinophenylmethylideneamino, tautomers thereof, or a salt thereof, wherein correspondingly substituted starting compounds are used.

**8.** A process according to claim 1 for the preparation of a compound of formula I wherein A is a single bond or an NH group; each of $X_1$ and $X_2$, independently of the other, is CH or N, with the proviso that at least one of the groups $X_1$ and $X_2$ is CH; $X_3$ and $X_4$ are CH; Y is NH or also, if A is a single bond, is O; Z is NH or S; the radical $R_3$ is hydrogen or lower alkyl; $R_1$, $R_2$, $R_4$ and $R_8$ are hydrogen, and $R_8$ is hydrogen, lower alkyl, hydroxy or amino, tautomers thereof, or a salt thereof, wherein correspondingly substituted starting compounds are used.

**9.** A process according to claim 1 for the preparation of 3-formylbenzamidine-2′-amidinohydrazone or a pharmaceutically acceptable salt thereof, wherein correspondingly substituted starting compounds are used.

**10.** A process according to claim 1 for the preparation of 3-acetylbenzamidine-2′-amidinohydrazone or a pharmaceutically acceptable salt thereof, wherein correspondingly substituted starting compounds are used.

**11.** A process according to claim 1 for the preparation of 1,3-bis(3′-amidinobenzylidene-amino)guanidine or a pharmaceutically acceptable salt thereof, wherein correspondingly substituted starting compounds are used.

12. A process according to claim 1 for the preparation of 2-amidino-6-formylpyridine-2′-amidinohydrazone or a pharmaceutically acceptable salt thereof, wherein correspondingly substituted starting compounds are used.

13. A process according to claim 1 for the preparation of 1-hydroxy-3-(2′-amidino-6′-pyridylmethylidene-amino)guanidine or a pharmaceutically acceptable salt thereof, wherein correspondingly substituted starting compounds are used.

14. A process according to claim 1 for the preparation of 3-formylbenzamidine-2′-(N-hydroxyamidino)hydrazone or a pharmaceutically acceptable salt thereof, wherein correspondingly substituted starting compounds are used.

15. A process for the preparation of a pharmaceutical composition comprising a compound of formula I prepared according to a process as claimed in any one of claims 1 to 14 and at least one pharmaceutically acceptable carrier, wherein such a compound of formula I is mixed with at least one pharmaceutically acceptable carrier.

## Revendications

### Revendications pour les Etats contractants suivants : AT, BE, CH, DE, FR, GB, IT, LI, LU, NL, SE

1. Composés de formule I

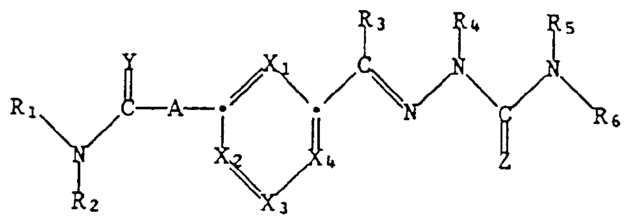

(I),

où A représente une liaison simple ou un groupe $NR_7$; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_8$ ou également O, dans le cas où A représente une liaison simple ; Z représente $NR_9$, O ou S ; $R_1$ représente l'hydrogène, un alkyle inférieur, l'hydroxy, un hydroxy éthéré ou estérifié ou un amino non substitué ou mono- ou disubstitué ; les restes $R_2$, $R_3$, $R_4$, $R_5$, $R_7$, $R_8$ et $R_9$ représentent, indépendamment les uns des autres, l'hydrogène ou un alkyle inférieur ou $R_1$ et $R_8$ peuvent également représenter ensemble un alkylène ; et $R_8$ représente l'hydrogène, un alkyle inférieur, un cycloalkyle, un arylalkyle inférieur, un aryle, un carboxy libre ou fonctionnellement modifié, un hydroxy, un hydroxy éthéré ou estérifié, un, amino non substitué ou mono- ou disubstitué, leurs tautomères et leurs sels.

2. Composés de formule I selon la revendication 1, où A représente une liaison simple ou un groupe $NR_7$ ; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_8$ ou également, dans le cas où A représente une liaison simple, O ; Z représente $NR_9$, O ou S: $R_1$ représente l'hydrogène, un alkyle inférieur, l'hydroxy, un alcoxy inférieur ou un amino ; les restes $R_2$, $R_3$, $R_4$, $R_5$, $R_7$, $R_8$ et $R_9$ représentent, indépendamment les uns des autres, l'hydrogène ou un alkyle inférieur ou $R_1$ et $R_8$ peuvent représenter ensemble également $-(CH_2)_2-$ ou $-(CH_2)_3-$ ; et $R_6$ représente l'hydrogène, un alkyle inférieur, l'hydroxy, un amino ou un groupe

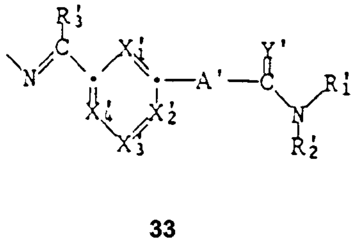

où A', X'$_1$, X'$_2$, X'$_3$, X'$_4$, Y', R'$_1$, R'$_2$ et R'$_3$ sont définis comme les restes A, $X_1$, $X_2$, $X_3$, $X_4$, Y, $R_1$, $R_2$ et $R_3$ correspondants, leurs tautomères et leurs sels.

3. Composés de formule I selon la revendication 1, où A représente une liaison simple ou un groupe NH ; les restes $X_1$, $X_2$, $X_3$ et $X_4$ ont les significations suivantes :
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_4 = N, X_1 = X_2 = X_3 = CH$
$X_1 = X_2 = N, X_3 = X_4 = CH$
$X_1 = X_3 = N, X_2 = X_4 = CH$
$X_1 = X_4 = N, X_2 = X_3 = CH$ ;
Y représente $NR_8$ ou également, lorsque A représente une liaison simple, O ; Z représente $NR_9$, O ou S; $R_1$ représente l'hydrogène, un alkyle inférieur, l'hydroxy, un alcoxy inférieur ou un amino ; les restes $R_2$, $R_3$, $R_8$ et $R_9$ représentent, indépendamment les uns des autres, l'hydrogène ou un alkyle inférieur ou $R_1$ et $R_8$ représentent ensemble également $-(CH_2)_2-$; $R_4$ et $R_5$ représentent l'hydrogène et $R_8$ représente l'hydrogène, un alkyle inférieur, l'hydroxy, un amino, le 3-amidinophénylméthylidèneamino ou le 2-amidino-6-pyridylméthylidèneamino, leurs tautomères et leurs sels.

4. Composés de formule I selon la revendication 1, où A représente une liaison simple ou un groupe NH ; où (a) $X_1$, $X_2$, $X_3$ et $X_4$ représentent CH ou (b) $X_1$ représente N et $X_2$, $X_3$ et $X_4$ représentent CH ou (c) $X_2$ représente N et $X_1$, $X_3$ et $X_4$ représentent CH ; ou (d)$X_1$ et $X_4$ représentent N et $X_2$ et $X_3$ représentent CH, Y représente $NR_8$ ou également, lorsque A représente une liaison simple, O ; Z représente NH ou S ; $R_1$ représente l'hydrogène, un alkyle inférieur, l'hydroxy, un alcoxy inférieur ou un amino ; les restes $R_2$, $R_3$ et $R_8$ représentent, indépendamment les uns des autres, l'hydrogène ou un alkyle inférieur ou $R_1$ et $R_8$ peuvent représenter ensemble également $-(CH_2)_2-$ ; $R_4$ et $R_8$ représentent l'hydrogène et $R_6$ représente l'hydrogène, un alkyle inférieur, l'hydroxy, un amino, le 3-amidinophénylméthylidèneamino ou le 2-amidino-6-pyridylméthylidèneamino, leurs tautomères et leurs sels.

5. Composés de formule I selon la revendication 1, où A représente une liaison simple ou un groupe NH ; où (a) $X_1$, $X_2$, $X_3$ et $X_4$ représentent CH ou (b) $X_1$ représente N et $X_2$, $X_3$ et $X_4$ représentent CH ou (c) $X_2$ représente N et $X_1$, $X_3$ et $X_4$ représentent CH ou (d) $X_1$ et X représentent N et $X_2$ et $X_3$ représentent CH ; Y représente NH ou également, lorsque A représente une liaison simple, O ; Z représente NH ou S ; $R_1$ représente l'hydrogène, un alkyle inférieur, l'hydroxy, un alcoxy inférieur ou un amino; $R_3$ représente l'hydrogène ou un alkyle inférieur; $R_2$, $R_4$ et $R_8$ représentent l'hydrogène et $R_8$ représente l'hydrogène, un alkyle inférieur, un hydroxy ou un amino, leurs tautomères et leurs sels.

6. Composés de formule I selon la revendication 1, où A représente une liaison simple ou un groupe $NR_7$ ; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_8$ ou également, lorsque A représente une liaison simple, O ; Z représente $NR_9$, O ou S; les restes $R_1$, $R_2$, $R_3$, $R_4$, $R_8$, $R_7$, $R_8$ et $R_9$ représentent, indépendamment les uns des autres, l'hydrogène ou un alkyle inférieur et $R_8$ représente l'hydrogène, un alkyle inférieur, un hydroxy, un amino ou le 3-amidinophénylmethylidèneamino, leurs tautomères ou leurs sels.

7. Composés de formule I selon la revendication 1, où A représente une liaison simple ou un groupe NH ; les restes $X_1$, $X_2$, $X_3$ et $X_4$ ont les significations suivantes :
$X_1 = X_2 = X_3 = X_4 = CH$
$X_1 = N, X_2 = X_3 = X_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$ ;
Y représente $NR_8$ ou également, lorsque A représente une liaison simple, O ; Z représente $NR_9$, O ou S; les restes $R_1$, $R_2$, $R_3$, $R_8$ et $R_9$ représentent, indépendamment les uns des autres, l'hydrogène ou un alkyle inférieur, $R_4$ et $R_5$ représentent l'hydrogène et $R_8$ représente l'hydrogène, un alkyle inférieur, l'hydroxy, un amino ou le 3-amidinophénylméthylidèneamino, leurs tautomères et leurs sels.

8. Composés de formule I selon la revendication 1, où A représente une liaison simple ou un groupe NH ; $X_1$ et $X_2$ représentent, indépendamment l'un de l'autre, CH ou N, sous réserve qu'au moins l'un des groupes $X_1$ et $X_2$ représente CH ; $X_3$ et $X_4$ représentent CH ; Y représente NH ou également, lorsque A représente une liaison simple, O ; Z représente NH ou S ; le reste $R_3$ représente l'hydrogène ou un alkyle inférieur ; $R_1$, $R_2$, $R_4$ et $R_5$ représentent l'hydrogène et $R_5$ représente l'hydrogène, un alkyle inférieur, l'hydroxy ou un amino, leurs tautomères et leurs sels.

9. La 3-formylbenzamidine-2′-amidinohydrazone selon la revendication 1 ou l'un de ses sels pharmaceutiquement utilisables.

10. La 3-acétylbenzamidine-2′-amidinohydrazone selon la revendication 1 ou l'un de ses sels pharmaceutiquement utilisables.

11. La 1,3-bis(3′-amidinobenzylidène-amino)guanidine selon la revendication 1 ou l'un de ses sels pharmaceutiquement utilisables.

12. La 2-amidino-6-formylpyridine-2′-amidinohydrazone selon la revendication 1 ou l'un de ses sels pharmaceutiquement utilisables.

13. La 1-hydroxy-3-(2′-amidino-6′-pyridylmethylidène-amino)-guanidine selon la revendication 1 ou l'un de ses sels pharmaceutiquement utilisables.

14. La 3-formylbenzamidine-2′-(N-hydroxyamidino)-hydrazone selon la revendication 1 ou l'un de ses sels pharmaceutiquement utilisables.

15. Préparations pharmaceutiques contenant un composé selon l'une des revendications 1 à 14 et au moins un support pharmaceutiquement utilisable.

16. Un composé selon l'une des revendications 1 à 14 destiné à être utilisé dans un procédé pour le traitement thérapeutique du corps animal ou humain.

17. Utilisation d'un composé selon l'une des revendications 1 à 14 pour l'obtention d'une préparation pharmaceutique destinée à servir dans un procéde pour le traitement thérapeutique des tumeurs.

18. Procéde pour la préparation d'un composé de formule I selon la revendication 1, caractérisé
a) en ce que l'on condense un composé de formule II

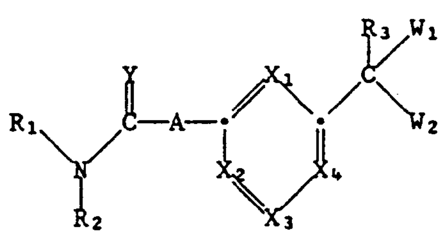

(II)

où le groupe $CW_1W_2$ représente un carbonyle, un carbonyle fonctionnellement modifié ou un carbonyle protégé, avec une amine de formule III

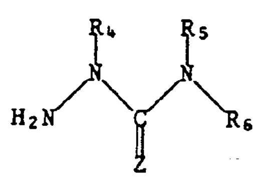

(III)

ou
b) en ce que, pour la préparation d'un compose de formule I où A représente un groupe $-NR_7-$, l'on fait réagir un composé de formule IV

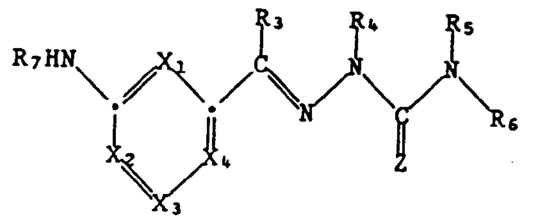

(IV)

avec un réactif approprié à transformer le groupe $-NHR_7$ en une guanidine de formule I ou

c) en ce que, pour la préparation d'un composé de formule I où A représente une liaison simple, l'on transforme dans un composé de formule VI

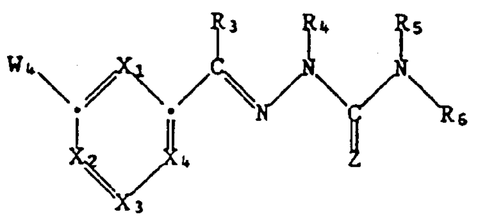

(VI)

où $W_4$ représente un reste pouvant être transformé en une amidine ou en un composé carbamoyle de formule I, le reste $W_4$ en un groupe $-C(=Y)NR_1R_2$ ; les symboles A, $X_1$, $X_2$, $X_3$, $X_4$, Y, Z et $R_1$-$R_7$ dans les produits de départ de formules II à VI ci-dessus ayant les significations données sous la formule I ; et, si désiré, en ce que l'on transforme le composé de formule I obtenu en un autre composé de formule I et/ou, si désiré, en ce que l'on transforme le sel obtenu en un composé libre ou en un autre sel et/ou, si desiré, en ce que l'on transforme le composé libre de formule I obtenu ayant des propriétés salifiables en un sel.

**Revendications pour les Etats contractants suivants : ES, GR**

1. Procéde pour la préparation des composés de formule I

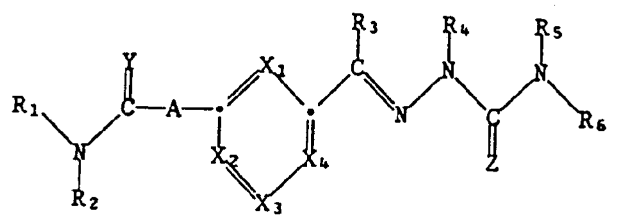

(I),

où A représente une liaison simple ou un groupe $NR_7$ ; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_8$ ou également O, dans le cas où A représente une liaison simple ; Z représente $NR_9$, O ou S ; $R_1$ représente l'hydrogène, un alkyle inférieur, l'hydroxy, un hydroxy étheré ou estérifié ou un amino non substitué ou mono- ou disubstitué ; les restes $R_2$, $R_3$, $R_4$, $R_8$, $R_7$, $R_8$ et $R_9$ représentent, indépendamment les uns des autres, l'hydrogène ou un alkyle inférieur ou $R_1$ et $R_8$ peuvent également représenter ensemble un alkylène ; et $R_6$ représente l'hydrogène, un alkyle inférieur, un cycloalkyle, un arylalkyle inférieur, un aryle, un carboxy libre ou fonctionnellement modifié, un hydroxy, un hydroxy éthéré ou esterifié, un amino non substitué ou mono- ou disubstitué, de leurs tautomères et de leurs sels, caractérisé

a) en ce que l'on condense un composé de formule II

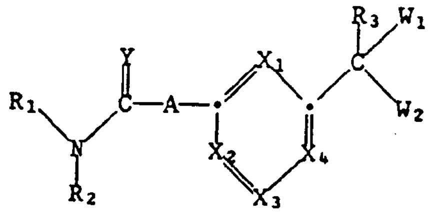

(II)

où le groupe $CW_1W_2$ représente un carbonyle, un carbonyle fonctionnellement modifié ou un carbonyle protégé, avec une amine de formule III

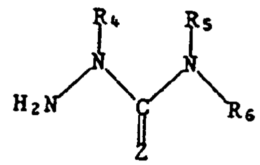

(III)

ou

b) en ce que, pour la préparation d'un composé de formule I où A représente un groupe $-NR_7-$, l'on fait réagir un composé de formule IV

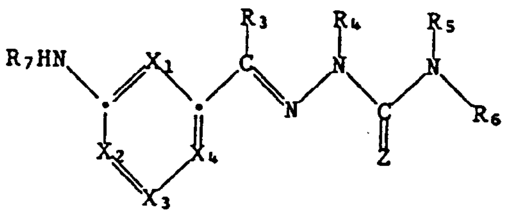

(IV)

avec un réactif approprié à transformer le groupe $-NHR_7$ en une guanidine de formule I ou

c) en ce que, pour la préparation d'un composé de formule I où A représente une liaison simple, l'on transforme dans un composé de formule VI

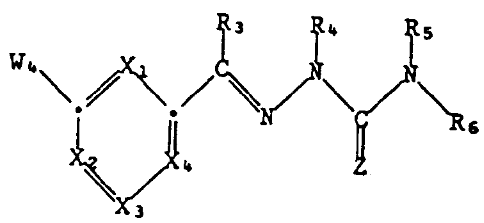

(VI)

où $W_4$ représente un reste pouvant être transformé en une amidine ou en un composé carbamoyle de formule I, le reste $W_4$ en un groupe $-C(=Y)NR_1R_2$ ; les symboles A, $X_1$, $X_2$, $X_3$, $X_4$, Y, Z et $R_1$-$R_7$ dans des produits de départ de formules II à VI ci-dessus ayant les significations données sous la formule I ; et, si desiré, en ce que l'on transforme le composé de formule I obtenu en un autre composé de formule I et/ou, si désiré, en ce que l'on transforme le sel obtenu en un composé libre ou en un autre sel et/ou, si désiré, en ce que l'on transforme le composé libre de formule I obtenu ayant des propriétés salifiables en un sel.

**2.** Procédé selon la revendication 1 pour la préparation des composés de formule I où A représente une liaison simple ou un groupe $NR_7$ ; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_8$ ou également, dans le cas où A représente une liaison simple, O ; Z représente $NR_9$, O ou S : $R_1$ représente l'hydrogène, un alkyle inférieur, l'hydroxy, un alcoxy inférieur ou un amino ; les restes $R_2$, $R_3$, $R_4$, $R_8$, $R_7$, $R_8$ et $R_9$ représentent, indépendamment les uns des autres, l'hydrogène ou un alkyle inférieur ou $R_1$ et $R_8$ peuvent

représenter ensemble également $-(CH_2)_2-$ ou $-(CH_2)_3-$ ; et $R_6$ représente l'hydrogène, un alkyle inférieur, l'hydroxy, un amino ou un groupe

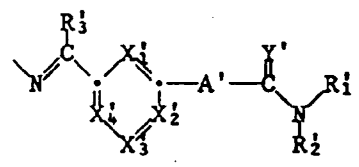

où A', $X'_1$, $X'_2$, $X'_3$, $X'_4$, Y', $R'_1$, $R'_2$ et $R'_3$ sont définis comme les restes A, $X_1$, $X_2$, $X_3$, $X_4$, Y, $R_1$, $R_2$ et $R_3$ correspondants, de leurs tautomères et de leurs sels, caractérisé en ce que l'on utilise des composés de départ substitués de façon correspondante.

3. Procédé selon la revendication 1 pour la préparation des composés de formule I où A représente une liaison simple ou un groupe NH : les restes $X_1$, $X_2$, $X_3$ et $X_4$ ont les significations suivantes:
$X_1 = X_2 = X_3 = X_4 = CH$
$X_1 = N$, $X_2 = X_3 = X_4 = CH$
$X_2 = N$, $X_1 = X_3 = X_4 = CH$
$X_3 = N$, $X_1 = X_2 = X_4 = CH$
$X_4 = N$, $X_1 = X_2 = X_3 = CH$
$X_1 = X_2 = N$, $X_3 = X_4 = CH$
$X_1 = X_3 = N$, $X_2 = X_4 = CH$
$X_1 = X_4 = N$, $X_2 = X_3 = CH$ ;
Y représente $NR_6$ ou également, lorsque A représente une liaison simple, O ; Z représente $NR_9$, O ou S; $R_1$ représente l'hydrogène, un alkyle inférieur, l'hydroxy, un alcoxy inférieur ou un amino ; les restes $R_2$, $R_3$, $R_6$ et $R_9$ représentent, indépendamment les uns des autres, l'hydrogène ou un alkyle inférieur ou $R_1$ et $R_8$ représentent ensemble également $-(CH_2)_2-$; $R_4$ et $R_5$ représentent l'hydrogène et $R_6$ représente l'hydrogène, un alkyle inférieur, l'hydroxy, un amino, le 3-amidinophenylmethylidèneamino ou le 2-amidino-6-pyridylmethylidèneamino, de leurs tautomères et de leurs sels, caractérisé en ce que l'on utilise des composés de départ substitués de façon correspondante.

4. Procédé selon la revendication 1 pour la préparation des composés de formule I où A représente une liaison simple ou un groupe NH ; où (a) $X_1$, $X_2$, $X_3$ et $X_4$ représentent CH ou (b) $X_1$ représente N et $X_2$, $X_3$ et $X_4$ représentent CH ou (c) $X_2$ représente N et $X_1$, $X_3$ et $X_4$ représentent CH ou (d) $X_1$ et $X_4$ représentent N et $X_2$ et $X_3$ représentent CH ; Y représente $NR_6$ ou également, lorsque A représente une liaison simple, O ; Z représente NH ou S ; $R_1$ représente l'hydrogène, un alkyle inférieur, l'hydroxy, un alcoxy inférieur ou un amino ; les restes $R_2$, $R_3$ et $R_6$ représentent, indépendamment les uns des autres, l'hydrogène ou un alkyle inférieur ou $R_1$ et $R_8$ peuvent représenter ensemble également $-(CH_2)2-$ ; $R_4$ et $R_6$ représentent l'hydrogène et $R_6$ représente l'hydrogène, un alkyle inférieur, l'hydroxy, un amino, le 3-amidinophénylméthylidèneamino ou le 2-amidino-6-pyridylméthylidèneamino, de leurs tautomères et de leurs sels, caractérisé en ce que l'on utilise des composés de départ substitués de façon correspondante.

5. Procédé selon la revendication 1 pour la préparation des composés de formule I où A représente une liaison simple ou un groupe NH ; où (a) $X_1$, $X_2$, $X_3$ et $X_4$ représentent CH ou (b) $X_1$ représente N et $X_2$, $X_3$ et $X_4$ représentent CH ou (c) $X_2$ représente N et $X_1$, $X_3$ et $X_4$ représentent CH ou (d) $X_1$ et $X_4$ représentent N et $X_2$ et $X_3$ représentent CH ; Y représente NH ou également, lorsque A représente une liaison simple, O ; Z représente NH ou S ; $R_1$ représente l'hydrogène, un alkyle inférieur, l'hydroxy, un alcoxy inférieur ou un amino ; $R_3$ représente l'hydrogène ou un alkyle inférieur ; $R_2$, $R_4$ et $R_5$ représentent l'hydrogène et $R_6$ représente l'hydrogène ou un alkyle inférieur, un hydroxy ou un amino, de leurs tautomères et de leurs sels, caracterisé en ce que l'on utilise des composés de départ substitués de façon correspondante.

6. Procédé selon la revendication 1 pour la preparation des composés de formule I où A représente une liaison simple ou un groupe $NR_7$ ; $X_1$, $X_2$, $X_3$ et $X_4$ représentent, indépendamment les uns des autres, CH ou N, sous réserve qu'au moins deux des groupes $X_1$-$X_4$ représentent CH ; Y représente $NR_6$ ou également, lorsque A représente une liaison simple, O ; Z représente $NR_9$, O ou S ; les restes $R_1$, $R_2$, $R_3$, $R_4$, $R_5$, $R_7$, $R_8$ et $R_9$ représentent, indépendamment les uns des autres, l'hydrogène ou un alkyle inférieur et $R_6$ représente l'hydrogène, un alkyle inférieur, un hydroxy, un amino ou le 3-amidinophénylméthylidèneamino,

de leurs tautomères ou de leurs sels, caractérisé en ce que l'on utilise des composés de départ substitués de façon correspondante.

7. Procédé selon la revendication 1 pour la préparation des composés de formule I où A représente une liaison simple ou un groupe NH ; les restes $X_1$, $X_2$, $X_3$ et $X_4$ ont les significations suivantes:
$X_1 = X_2 = X_3 = X_4 = CH$
$X_1 = N, X_2 = X_3 = C_4 = CH$
$X_2 = N, X_1 = X_3 = X_4 = CH$
$X_3 = N, X_1 = X_2 = X_4 = CH$
$X_1 = X_2 = N, X_3 = X_4 = CH$
$X_1 = X_3 = N, X_2 = X_4 = CH$
$X_1 = X_4 = N, X_2 = X_3 = CH$ ;
Y représente $NR_8$ ou également, lorsque A représente une liaison simple, O ; Z représente $NR_9$, O ou S; les restes $R_1$, $R_2$, $R_3$, $R_8$ et $R_9$ représentent, indépendamment les uns des autres, l'hydrogène ou un alkyle inférieur, $R_4$ et $R_5$ représentent l'hydrogène et $R_8$ représente l'hydrogène, un alkyle inférieur, l'hydroxy, un amino ou le 3-amidinophénylméthylidèneamino, de leurs tautomères et de leurs sels, caractérisé en ce que l'on utilise des composés de départ substitués de façon correspondante.

8. Procédé selon la revendication 1 pour la préparation des composés de formule I où A représente une liaison simple ou un groupe NH ; $X_1$ et $X_2$ représentent, indépendamment l'un de l'autre, CH ou N, sous réserve qu'au moins l'un des groupes $X_1$ et $X_2$ représente CH ; $X_3$ et $X_4$ représentent CH ; Y représente NH ou également, lorsque A représente une liaison simple, O ; Z représente NH ou S ; le reste $R_3$ représente l'hydrogène ou un alkyle inférieur ; $R_1$, $R_2$, $R_4$ et $R_8$ représentent l'hydrogène et $R_8$ représente l'hydrogène, un alkyle inférieur, l'hydroxy ou un amino, de leurs tautomères et de leurs sels, caractérisé en ce que l'on utilise des composés de départ substitués de façon correspondante.

9. Procédé selon la revendication 1 pour la préparation de la 3-formylbenzamidine-2′-amidinohydrazone ou de l'un de ses sels pharmaceutiquement utilisables, caractérisé en ce que l'on utilise des composés de départ substitués de façon correspondante.

10. Procédé selon la revendication 1 pour la préparation de le 3-acétylbenzamidine-2′-amidinohydrazone ou de l'un de ses sels pharmaceutiquement utilisables, caractérisé en ce que l'on utilise des composés de départ substitués de façon correspondante.

11. Procédé selon la revendication 1 pour la préparation de la 1,3-bis(3′-amidinobenzylidèneamino)-guanidine ou de l'un de ses sels pharmaceutiquement utilisables, caractérisé en ce que l'on utilise des composés de départ substitués de façon correspondante.

12. Procédé selon la revendication 1 pour la préparation de la 2-amidino-6-formylpyridine-2′-amidinohydrazone ou de l'un de ses sels pharmaceutiquement utilisables, caractérisé en ce que l'on utilise des composés de départ substitués de façon correspondante.

13. Procédé selon la revendication 1 pour la préparation de la 1-hydroxy-3-(2′-amidino-6′-pyridylméthylidène-amino)-guanidine ou de l'un de ses sels pharmaceutiquement utilisables, caractérisé en ce que l'on utilise des composés de départ substitués de façon correspondante.

14. Procédé selon la revendication 1 pour la préparation de la 3-formylbenzamidine-2′-(N-hydroxyamidino)-hydrazone ou de l'un de ses sels pharmaceutiquement utilisables, caractérisé en ce que l'on utilise des composés de départ substitués de façon correspondante.

15. Procédé pour l'obtention de préparations pharmaceutiques contenant un composé de formule I préparé selon un procédé de l'une des revendications 1 à 14 et au moins un support pharmaceutiquement utilisable, caractérisé en ce que l'on mélange un tel composé de formule I avec au moins un support pharmaceutiquement utilisable.